# EUROPEAN PATENT SPECIFICATION

(11) **EP 4 135 625 B1**
(45) Date of publication and mention of the grant of the patent: **12.08.2026**
(21) Application number: 21720957.6
(22) Date of filing: 12.04.2021
(51) Int. Cl.: A61F 2/24

(54) **ENDOVASCULAR DELIVERY APPARATUS HAVING VARIABLE LENGTH BALLOON**
ENDOVASKULÄRE ABGABEVORRICHTUNG MIT VARIABLER BALLONLÄNGE
APPAREIL D'ADMINISTRATION ENDOVASCULAIRE AYANT UN BALLONNET À LONGUEUR VARIABLE

(30) Priority: 13.04.2020 US 202063009072 P
(43) Date of publication of application: 22.02.2023
(73) Proprietor: Edwards Lifesciences Corporation, Irvine, CA 92614 (US)
(72) Inventor: HICKS, Kristen, Irvine, CA 92614 (US); MURAD, Michael, C., Irvine, CA 92614 (US); BIALAS, Michael, R., Irvine, CA 92614 (US); LEE, Walter, Irvine, CA 92614 (US)
(74) Representative: Eisenführ Speiser
(86) International application number: PCT/US2021/026802
(87) International publication number: WO 2021/211410

(56) References cited:
- WO-A1-2017/040823
- US-A1- 2007 088 431
- US-A1- 2008 065 011
- US-A1- 2013 345 787
- US-B2- 9 061 119

## Description

### CROSS-REFERENCE TO RELATED APPLICATION

The present application claims the benefit of U.S. Provisional Application No. 63/009,072, filed April 13, 2020.

### FIELD

The present disclosure concerns embodiments of an endovascular delivery apparatus, such as for implanting a prosthetic heart valve or other implantable medical devices, wherein the delivery apparatus has a variable length balloon.

### BACKGROUND

Endovascular delivery devices are used in various procedures to deliver prosthetic medical devices or instruments to locations inside the body that are not readily accessible by surgery or where access without surgery is desirable. Access to a target location inside the body can be achieved by inserting and guiding the delivery device through a pathway or lumen in the body, including, but not limited to, a blood vessel, an esophagus, a trachea, any portion of the gastrointestinal tract, a lymphatic vessel, to name a few. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size such as by inflating a balloon on which the prosthetic valve is mounted, or by deploying the prosthetic valve from a sheath of the delivery device so that the prosthetic valve can self-expand to its functional size.

Balloon-expandable prosthetic valves may be preferred for replacing calcified native valves because the catheter balloon can apply a sufficient expansion force to expand and anchor the frame of the prosthetic valve against the surrounding calcified tissue. In one known technique for delivering a prosthetic heart valve, the prosthetic heart valve may be crimped on a valve retaining portion of a balloon of the delivery catheter prior to insertion into the patient's body. Upon reaching the target site, the balloon is inflated to deliver the prosthetic valve. Then, the balloon is deflated so that the catheter can be removed from the patient's body.

Typically, the distal end of the balloon is mounted on an inner shaft of the delivery catheter and the proximal end of the balloon is mounted to an outer shaft of the delivery catheter. A proximal end of the inner shaft is fixed at a designated location along the delivery catheter (e.g., at an inflation hub of the catheter). During the manufacturing process, the balloon can be tightly folded into a compact, deflated state to minimize the overall crimp profile of the delivery apparatus when the prosthetic valve is crimped onto the balloon. However, after the prosthetic valve is expanded in a patient's body and the balloon is deflated, the balloon may not refold efficiently, if at all. This can result in high retrieval forces when the delivery catheter is withdrawn from the patient's body via an introducer sheath, making removal of the delivery catheter difficult.

Accordingly, there exists a need for new and improved delivery catheters for prosthetic valves.

US 2008/0065011 A1 discloses a delivery apparatus that includes a balloon catheter having an inflatable balloon which mounts a crimped valve for delivery through the patient's vasculature. The delivery apparatus can include a guide, or flex, catheter having a shaft that extends over the shaft of the balloon catheter. The guide catheter shaft has a steerable section, the curvature of which can be adjusted by the operator to facilitate navigation of the delivery apparatus around bends in the vasculature. The delivery apparatus also can include a nose catheter having a shaft that extends through the balloon catheter shaft and a nose piece located distally of the valve. The nose piece desirably has a tapered outer surface and is made of a flexible material to provide atraumatic tracking through the arteries and a stenotic native valve. The nose piece desirably has an internal bore that is dimensioned to receive at least a distal end portion of the deflated balloon during delivery of the valve.

### SUMMARY

Disclosed herein are balloon catheters that can be used to deliver a medical device, tools, agents, or other therapy to a location within a body of a subject. Also disclosed are methods of using the balloon catheter for delivering the therapy to a target location within a body of a subject. In some embodiments, balloon catheters can be used to deliver an implantable medical device, such as a prosthetic heart valve, to a target site in a patient, such as a heart. In some embodiments, balloon catheters can be a component of a delivery system (e.g., an endovascular or transcatheter delivery system) that can be used to deliver a prosthetic heart valve or other implantable medical device.

In one representative embodiment, a delivery apparatus for an implantable prosthetic device comprises a handle; a first shaft extending distally from the handle; a second shaft extending distally from the handle, wherein the first shaft extends through the second shaft and is movable axially relative to the second shaft; an inflatable balloon having a proximal end portion and a distal end portion, wherein the proximal end portion of the balloon is coupled to a distal end portion of the second shaft and the distal end portion of the balloon is coupled to a distal end portion of the first shaft; and an inflation hub assembly comprising an inflation manifold and a piston, wherein the inflation manifold comprises a main body defining a main lumen extending therethrough and an inflation port defining an inflation port lumen in fluid communication with the main lumen, wherein the piston extends into the main lumen and is slidable relative to the inflation manifold; wherein a proximal end portion of the first shaft is coupled to the piston and a proximal end portion of the second shaft is coupled to the main body of the inflation manifold or the handle; wherein the piston is moveable relative to the inflation manifold in proximal and distal directions such that distal movement of the piston produces distal movement of the first shaft relative to the second shaft, which increases the length of the balloon, and such that proximal movement of the piston produces proximal movement of the first shaft relative to the second shaft, which decreases the length of the balloon.

In another representative embodiment, a delivery apparatus for an implantable prosthetic device comprises a handle; a first shaft extending distally from the handle; a second shaft extending distally from the handle, wherein the first shaft extends through the second shaft and is movable axially relative to the second shaft in proximal and distal directions; and an inflatable balloon having a proximal end portion and a distal end portion, wherein the proximal end portion of the balloon is coupled to a distal end portion of the second shaft and the distal end portion of the balloon is coupled to a distal end portion of the first shaft; wherein the first shaft is fixed against rotational movement relative to the second shaft and wherein distal movement of the first shaft relative to the second shaft moves the distal end portion of the balloon away from the proximal end portion of the balloon to increase a length of the balloon and proximal movement of the first shaft relative to the second shaft moves the distal end portion of the balloon toward the proximal end portion of the balloon to decrease the length of the balloon.

**In** another representative embodiment, a delivery apparatus for an implantable prosthetic device comprises a handle; a first shaft extending distally from the handle; a second shaft extending distally from the handle, wherein the first shaft extends through the second shaft and is movable axially relative to the second shaft in proximal and distal directions; a biasing member configured to apply a biasing force that biases the first shaft to move in the distal direction relative to the second shaft; and an inflatable balloon having a proximal end portion and a distal end portion, wherein the proximal end portion of the balloon is coupled to a distal end portion of the second shaft and the distal end portion of the balloon is coupled to a distal end portion of the first shaft; wherein distal movement of the first shaft relative to the second shaft under the biasing force moves the distal end portion of the balloon away from the proximal end portion of the balloon to increase a length of the balloon and proximal movement of the first shaft relative to the second shaft against the biasing force moves the distal end portion of the balloon toward the proximal end portion of the balloon to decrease the length of the balloon.

**In** another representative embodiment, a method of implanting a prosthetic heart valve using a delivery apparatus is provided, wherein the delivery apparatus can be any of the embodiments described above. The method comprises delivering the prosthetic heart valve to a patient's heart while the prosthetic heart valve is in a radially compressed state on the balloon of the delivery apparatus; inflating the balloon to radially expand the prosthetic heart against surrounding tissue within the heart; after inflating the balloon to radially the prosthetic heart valve, deflating the balloon; and increasing the length of the balloon during or after the act of deflating the balloon.

**In** another representative embodiment, a method of implanting a prosthetic heart valve comprises inserting the distal end portion of a delivery apparatus and a prosthetic heart valve into the vasculature of a patient, wherein the prosthetic heart valve is in a radially compressed on a balloon of the delivery apparatus, wherein the delivery apparatus comprises a first shaft and a second shaft, the first shaft extending through the first shaft, and wherein a proximal end portion of the balloon is coupled to a distal end portion of the second shaft and a distal end portion of the balloon is coupled to a distal end portion of the first shaft; advancing the prosthetic heart valve to an implantation location in the heart; inflating the balloon to radially expand the prosthetic heart against surrounding tissue within the heart; after inflating the balloon to radially the prosthetic heart valve, deflating the balloon; and while deflating the balloon, moving the first shaft distally relative to the second to increase the length of the balloon.

The foregoing and other objects, features, and advantages of the invention, the invention being defined in the appended claims, will become more apparent from the following detailed description, which proceeds with reference to the accompanying figures.

### BRIEF DESCRIPTION OF THE DRAWINGS

FIG. 1 is a perspective view of an embodiment of a prosthetic heart valve that can be implanted using any of the delivery apparatuses disclosed herein.
FIG. 2A is a perspective view of another embodiment of a prosthetic heart valve that can be implanted using any of the delivery apparatuses disclosed herein.
FIG. 2B is a perspective view of the prosthetic heart valve of FIG. 2A with the components on the outside of the frame shown in transparent lines for purpose of illustration.
FIG. 3 is a perspective view of a delivery apparatus for a prosthetic heart valve, according to an embodiment.
FIG. 4 is a cross-sectional view of an embodiment of a distal end portion of the delivery apparatus of FIG. 3.
FIG. 5 is a side view of the distal end portion of the delivery apparatus of FIG. 3, shown with a prosthetic heart valve mounted in a radially crimped state on the balloon of the delivery apparatus.
FIG. 6 is a cross-sectional view of a handle of a delivery apparatus, according to one embodiment.
FIG. 7 is a cross-sectional view of a handle of a delivery apparatus, according to another embodiment.
FIG. 8A is a side view of the inflation hub assembly of the delivery apparatus of FIG. 7.
FIG. 8B is a side cross-sectional view of the inflation hub assembly of FIG. 8A.
FIG. 9A is a side view of an inflation manifold of the inflation hub assembly shown in FIGS. 8A-8B.
FIG. 9B is a side cross-sectional view of the inflation manifold of FIG. 9A.
FIG. 10A is a side view of a piston of the inflation hub assembly shown in FIGS. 8A-8B.
FIG. 10B is a side cross-sectional view of the piston of FIG. 10A.
FIG. 11A is a perspective view of an embodiment of a cap member of the inflation hub assembly shown in FIGS. 8A-8B.
FIG. 11B is a side cross-sectional view of the cap member of FIG. 11A.
FIG. 12A is another side view of the inflation hub assembly of the delivery apparatus of FIG. 7.
FIG. 12B is a side cross-sectional view of the inflation hub assembly of FIG. 12A.
FIGS. 13A and 13B show the distal end portion and the proximal end portion, respectively, of the delivery apparatus of FIG. 7, when the piston (FIG. 13B) is in a proximal position and the balloon (FIG. 13A) is in a corresponding axially foreshortened state.
FIGS. 14A and 14B show the distal end portion and the proximal end portion, respectively, of the delivery apparatus of FIG. 7, when the piston (FIG. 14B) is in a distal position and the balloon (FIG. 14A) is in a corresponding axially elongated state.
FIG. 15 is a cross-sectional view of the distal end portion of a delivery apparatus, according to another embodiment.
FIG. 16 is a cross-sectional view of the proximal end portion of a delivery apparatus, according to another embodiment.
FIG. 17 is a flowchart of a method of implanting a prosthetic heart valve with a delivery apparatus having a balloon that is adjustable in length.
FIGS. 18A, 18B, and 18C show alternative embodiments of a piston for use in an inflation hub assembly.
FIGS. 19A and 19B show a delivery device for delivering a prosthetic heart valve, according to another embodiment.
FIG. 20 is a cross-sectional view of an inflation manifold, according to another embodiment, that can be implemented in any of the delivery devices disclosed herein.

### DETAILED DESCRIPTION

### General Considerations

For purposes of this description, certain aspects, advantages, and novel features of the examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

In the interest of conciseness, and for the sake of continuity in the description, same or similar reference characters may be used for same or similar elements in different figures, and description of an element in one figure will be deemed to carry over when the element appears in other figures with the same or similar reference character. In some cases, the term "corresponding to" may be used to describe correspondence between elements of different figures. In an example usage, when an element in a first figure is described as corresponding to another element in a second figure, the element in the first figure is deemed to have the characteristics of the other element in the second figure, and vice versa, unless stated otherwise.

The word "comprise" and derivatives thereof, such as "comprises" and "comprising", are to be construed in an open, inclusive sense, that is, as "including, but not limited to". The singular forms "a", "an", "at least one", and "the" include plural referents, unless the context dictates otherwise. The term "and/or", when used between the last two elements of a list of elements, means any one or more of the listed elements. The term "or" is generally employed in its broadest sense, that is, as meaning "and/or", unless the context clearly dictates otherwise.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

Described herein are examples of transcatheter delivery apparatuses and methods for using the same.

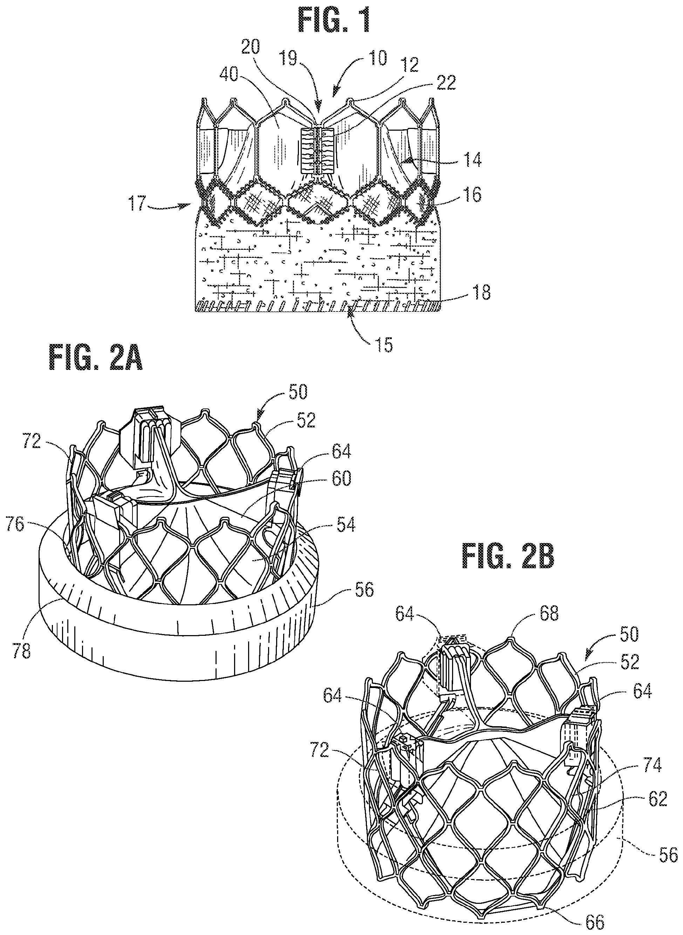

In some embodiments, a delivery apparatus is adapted to deliver a prosthetic heart valve crimped on the valve retaining portion of a balloon. FIG. 1 shows a prosthetic heart valve 10, according to one embodiment. The illustrated prosthetic valve is adapted to be implanted in the native aortic annulus, although in other embodiments it can be adapted to be implanted in the other native annuluses of the heart (e.g., the pulmonary, mitral, and tricuspid valves). The prosthetic valve can also be adapted to be implanted in other tubular organs or passageways in the body. The prosthetic valve 10 can have four main components: a stent or frame 12, a valvular structure 14, an inner skirt 16, and a perivalvular outer sealing member or outer skirt 18. The prosthetic valve 10 can have an inflow end portion 15, an intermediate portion 17, and an outflow end portion 19.

The valvular structure 14 can comprise three leaflets 40, collectively forming a leaflet structure, which can be arranged to collapse in a tricuspid arrangement, although in other embodiments there can be greater or fewer number of leaflets (e.g., one or more leaflets 40). The leaflets 40 can be secured to one another at their adjacent sides to form commissures 22 of the leaflet structure 14. The lower edge of valvular structure 14 can have an undulating, curved scalloped shape and can be secured to the inner skirt 16 by sutures (not shown). In some embodiments, the leaflets 40 can be formed of pericardial tissue (e.g., bovine pericardial tissue), biocompatible synthetic materials, or various other suitable natural or synthetic materials as known in the art and described in U.S. Patent No. 6,730,118.

The frame 12 can be formed with a plurality of circumferentially spaced slots, or commissure windows 20 that are adapted to mount the commissures 22 of the valvular structure 14 to the frame. The frame 12 can be made of any of various suitable plastically-expandable materials (e.g., stainless steel, etc.) or self-expanding materials (e.g., nickel titanium alloy (NiTi), such as nitinol), as known in the art. When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular embodiments, the frame 12 is made of a nickel-cobalt-chromium-molybdenum alloy, such as MP35N^{®} alloy (SPS Technologies, Jenkintown, Pennsylvania), which is equivalent to UNS R30035 alloy (covered by ASTM F562-02). MP35N^{®} alloy/UNS R30035 alloy comprises 35% nickel, 35% cobalt, 20% chromium, and 10% molybdenum, by weight. Additional details regarding the prosthetic valve 10 and its various components are described in WIPO Patent Application Publication No. WO 2018/222799.

FIG. 2A is a perspective view of a prosthetic heart valve 50, according to another embodiment. The valve 50 can have three main components: a stent or frame, 52, a valvular structure 54, and a sealing member 56. FIG. 2B is a perspective view of the prosthetic valve 50 with the components on the outside of the frame 52 (including the sealing member 56) shown in transparent lines for purposes of illustration.

Like the valvular structure 14 of FIG. 1, the valvular structure 54 can comprise three leaflets 60, collectively forming a leaflet structure, which can be arranged to collapse in a tricuspid arrangement. Each leaflet 60 can be coupled to the frame 52 along its inflow edge 62 (the lower edge in the figures; also referred to as "cusp edges") and at commissures 64 of the valvular structure 54 where adjacent portions of two leaflets are connected to each other. A reinforcing element (not shown), such as a fabric strip, can be connected directly to the cusp edges of the leaflets and to the struts of the frame to couple the cusp edges of the leaflets to the frame.

Similar to the frame 12 of FIG. 1, the frame 52 can be made of any of various suitable plastically-expandable materials or self-expanding materials, as known in the art and described above. The frame 52 in the illustrated embodiment comprises a plurality of circumferentially extending rows of angled struts 72 defining rows of cells, or openings, 74 of the frame. The frame 52 can have a cylindrical or substantially cylindrical shape having a constant diameter from an inflow end 66 to an outflow end 68 of the frame as shown, or the frame can vary in diameter along the height of the frame, as disclosed in U.S. Patent Publication No. 2012/0239142.

The sealing member 56 in the illustrated embodiment is mounted on the outside of the frame 52 and functions to create a seal against the surrounding tissue (e.g., the native leaflets and/or native annulus) to prevent or at least minimize paravalvular leakage. The sealing member 56 can comprise an inner layer 76 (which can be in contact with the outer surface of the frame 52) and an outer layer 78. The sealing member 56 can be connected to the frame 52 using suitable techniques or mechanisms. For example, the sealing member 56 can be sutured to the frame 52 via sutures that can extend around the struts 72 and through the inner layer 76. In alternative embodiments, the inner layer 76 can be mounted on the inner surface of the frame 52, while the outer layer 78 is on the outside of the frame 52.

The outer layer 78 can be configured or shaped to extend radially outward from the inner layer 76 and the frame 52 when the prosthetic valve 50 is deployed. When the prosthetic valve is fully expanded outside of a patient's body, the outer layer 78 can expand away from the inner layer 76 to create a space between the two layers. Thus, when implanted inside the body, this allows the outer layer 78 to expand into contact with the surrounding tissue.

Additional details regarding the prosthetic valve 50 and its various components are described in U.S. Patent Publication No. 2018/0028310.

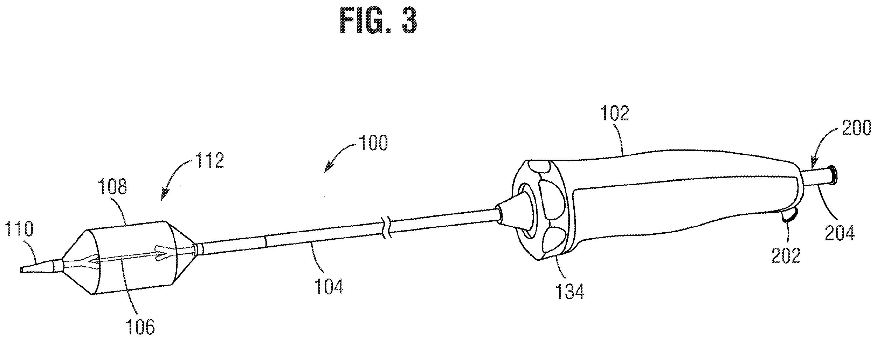

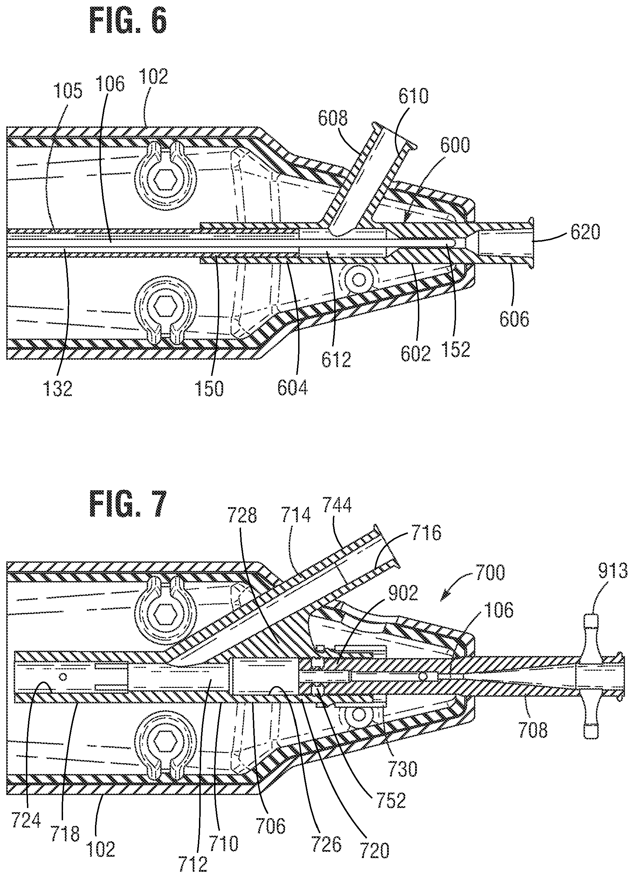

FIG. 3 shows a delivery apparatus (also referred to as a delivery device) 100, according to one embodiment, that can be used to implant an expandable prosthetic heart valve (e.g., heart valve 10 or 50), or another type of expandable prosthetic medical device (such as a stent). In some embodiments, the delivery apparatus 100 is specifically adapted for use in introducing a prosthetic valve into a heart of a patient. The delivery apparatus of FIG. 3 can include an inflation hub assembly in accordance with the present disclosure, as further described below with respect to FIGS. 7- 16.

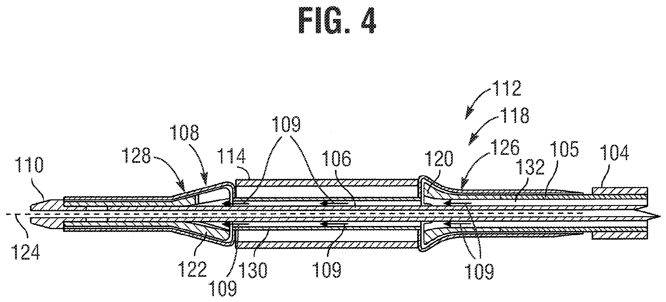

Referring to FIG. 3, the delivery apparatus 100 in the illustrated embodiment is a balloon catheter comprising a handle 102, a steerable, outer shaft 104 extending from the handle 102, an intermediate shaft 105 (see FIG. 4) extending from the handle 102 coaxially through the steerable outer shaft 104, and an inner shaft 106 extending from the handle 102 coaxially through the intermediate shaft 105 and the steerable shaft 104, an inflatable balloon 108 extending from a distal end of the intermediate shaft 105, and a nosecone 110 arranged at a distal end of the delivery apparatus 100. A distal end portion 112 of the delivery apparatus 100 includes the balloon 108, the nosecone 110, and a balloon shoulder assembly. A prosthetic medical device, such as a prosthetic heart valve may be mounted on a valve retaining portion of the balloon 108, as described further below with reference to FIG. 4. The balloon shoulder assembly may be configured to maintain the prosthetic heart valve or other medical device at a fixed position on the balloon 108 during delivery through the patient's vasculature.

The handle 102 can include a steering mechanism configured to adjust the curvature of the distal end portion of the delivery apparatus. In the illustrated embodiment, for example, the handle 102 includes an adjustment member, such as the illustrated rotatable knob 134, which in turn is operatively coupled to the proximal end portion of a pull wire (not shown). The pull wire extends distally from the handle 102 through the outer shaft 104 and has a distal end portion affixed to the outer shaft at or near the distal end of the outer shaft 104. Rotating the knob 134 is effective to increase or decrease the tension in the pull wire, thereby adjusting the curvature of the distal end portion of the delivery apparatus.

As further shown in FIG. 3, the delivery apparatus 100 can also include an inflation hub 200 at a proximal end of the delivery apparatus. The inflation hub 200 in the illustrated embodiment is partially housed within the handle 102 and includes an inflation port 202 and a proximal leg portion 204 extending out of the handle 102.

FIG. 4 shows an embodiment of the distal end portion 112 of the delivery apparatus 100. As shown in FIG. 4, the delivery apparatus 100 is configured to mount a prosthetic valve 114 (e.g., which can be prosthetic heart valve 10 or 50) in a crimped state over the balloon 108 for insertion of the delivery apparatus 100 and the prosthetic valve 114 into a patient's vasculature.

As shown in FIG. 4, at a proximal end of the distal end portion 112, the inner shaft 106 extends distally beyond the steerable shaft 104 and the intermediate shaft 105 and through the balloon 108. The balloon 108 can be supported on a balloon shoulder assembly 118. The balloon shoulder assembly 118 includes a proximal shoulder 120 connected to a distal end of the intermediate shaft 105 and a distal shoulder 122 mounted on the inner shaft 106. The balloon 108 includes a proximal end portion 126 surrounding and/or folded over the proximal shoulder 120 and a distal end portion 128 surrounding and/or folded over the distal shoulder 122. In some embodiments, the proximal end portion 126 of the balloon 108 may be secured to the outer surface of the intermediate shaft 105. In some embodiments, the distal end portion 128 of the balloon 108 may be secured to the outer surface of the nosecone 110, which can be mounted on or coupled to the inner shaft 106.

In the illustrated embodiment, the nosecone 110 and the distal shoulder 122 can be a one-piece or unitary component, that is, the nosecone 110 is a distal portion of the unitary component and the distal shoulder 122 is a proximal portion of the unitary component. In other embodiments, the nosecone 110 and the distal shoulder 122 can be separate components, and each can be mounted on the inner shaft 106 next to each other or at axially spaced locations.

The proximal shoulder 120 and the distal shoulder 122 are spaced apart from one another, in an axial direction relative to a central longitudinal axis 124 of the delivery apparatus 100. As a result, the balloon 108 defines a valve-retaining portion 130 in the space that separates the proximal shoulder 120 and the distal shoulder 122 (e.g., between flared ends of the proximal shoulder 120 and the distal shoulder 122). As shown in FIG. 4, the prosthetic valve 114 can be crimped onto the valve retaining portion 130 of the balloon 108, between the proximal shoulder 120 and the distal shoulder 122, thereby preventing or reducing axial movement of the prosthetic valve 114 relative to the balloon 108 during insertion of the delivery device 100 into the patient and delivery of the prosthetic valve 114 to the target implantation site. In alternative embodiments, the delivery apparatus 100 does not have the proximal and distal shoulders 120, 122, respectively.

The outer diameter of the inner shaft 106 can be sized such that an annular space 132 is defined between the inner shaft 106 and the intermediate shaft 105 along the entire length of the intermediate shaft 105. The annular space 132 may be fluidly coupled to one or more fluid passageways of the delivery apparatus 100 which can be fluidly connectable to a fluid source (e.g., a syringe) that can inject an inflation fluid (e.g., saline) into the delivery apparatus. In the illustrated embodiment, for example, the inflation port 202 of the inflation hub 200 can be in fluid communication with the annular space 132. In this way, an inflation fluid from the fluid source can flow through the inflation port 202, through the annular space 132, and into the balloon 108 to inflate the balloon 108 and expand and deploy the prosthetic valve 114.

FIG. 4 illustrates the flow of the inflation fluid (indicated by arrows 109) through the annular space 132 and through passages in the proximal shoulder 120 and distal shoulder 122. The fluid can then flow into the proximal and distal end portions 126, 128 of the balloon 108 to expand the valve 114. Further details of the balloon shoulder assembly, the steering mechanism, and other components of the delivery device are disclosed in U.S. Publication Nos. 2007/0005131, 2009/0281619, 2013/0030519, and 2017/0065415. Other examples of delivery devices for a prosthetic valve (e.g., valve 10 or 50) are disclosed in U.S. Application Nos. 63/069,567, filed August 24, 2020 and 63/138,890, filed January 19, 2021. Any of the devices described herein for adjusting the length of a balloon of a delivery device can be incorporated in the delivery devices disclosed in the previously mentioned applications.

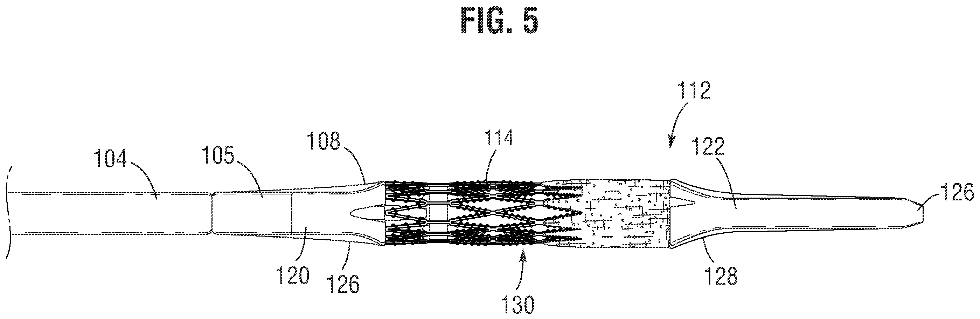

FIG. 5 shows a side view of an exterior of the distal end portion 112 of the delivery apparatus 100, including the prosthetic valve 114 crimped on the balloon 108. As shown in FIG. 5, the balloon 108 includes the proximal end portion 126 surrounding and/or folded over the proximal shoulder 120, the distal end portion 128 surrounding and/or folded over the distal shoulder 122, and the valve retaining portion 130 located between the proximal end portion 126 and the distal end portion 128.

FIG. 6 shows a cross-section of the handle 102 and a known inflation hub 600 partially housed in the handle 102. The inflation hub 600 includes a main body 602 in the form of a shaft, which includes a distal end portion 604 and a proximal end portion 606 extending outwardly from the handle 102. An inflation port 608 extends from the distal end portion 604 of the shaft 602 and outwardly through the handle.

A proximal end portion 150 of the intermediate shaft 105 extends into the distal end portion 604 of the shaft 602 and is fixed relative to the shaft, such as with an adhesive or welding. A proximal end portion 152 of the inner shaft 106 extends into the shaft 602, beyond the proximal end portion 150 of the intermediate shaft 105, and into a narrower bore region of the proximal end portion 606 of the shaft, where it is fixed relative to the shaft, such as with an adhesive or welding, creating a hermetic seal.

The inflation port 608 has an inflation lumen 610 that is in fluid communication with a main lumen 612 of the shaft 602, which in turn is in fluid communication with the annular space 132 between the inner shaft 106 and the intermediate shaft 105. In use, a source of an inflation fluid (e.g., a syringe) can be fluidly coupled to the inflation port 608, such as by connecting one end of a tube or conduit (e.g., flexible medical tubing) to the inflation port 608 and the other end of the tube to the source of the inflation fluid.

To inflate the balloon and deploy the prosthetic valve, the inflation fluid from the source is introduced into the inflation port 608, and flows through the inflation lumen 610, into the main lumen 612, through the annular space 132, and into the balloon. The proximal end portion 606 of the shaft 602 can have a proximal opening 620 through which a guidewire (not shown) can extend. In use, the delivery apparatus 100 can be advanced over the guidewire (previously inserted into the patient's vasculature), which extends through a guidewire lumen of the inner shaft 106, the shaft 602 of the hub 600, and outwardly through the proximal opening 620.

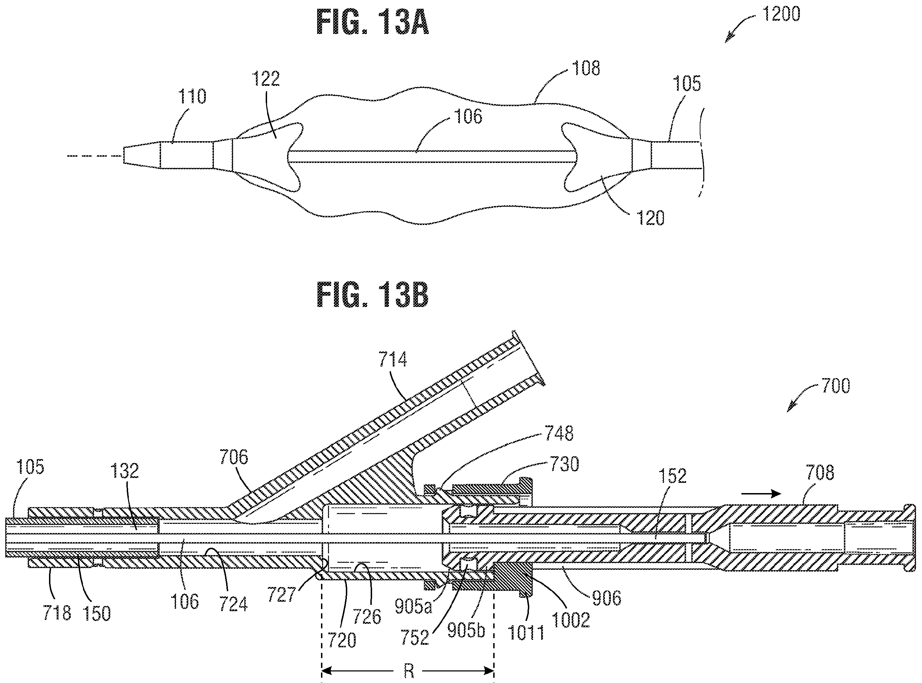

As discussed above in connection with FIG. 4, the proximal end portion 126 of the balloon 108 is connected to the intermediate shaft 105 and the distal end portion 128 of the balloon 108 is connected to the nosecone 110, which in turn is connected to the inner shaft 106, such as with an adhesive or welding, creating a hermetic seal. Due to the fixed positions of the proximal end portion 150 of the intermediate shaft 105 and the proximal end portion 152 of the inner shaft 106, the length of the balloon 108 is fixed. Prior to insertion into the patient's vasculature, the balloon 108 is deflated and folded into a compact and low profile, as best depicted in FIG. 4. However, after the balloon is deflated following deployment of a prosthetic device, such as a prosthetic heart valve, the balloon does not automatically revert back to its pre-deployed folded configuration. Consequently, the post-deployed, uninflated profile of the balloon is relatively large. An example of a larger than desired balloon profile is shown in FIG. 13A. Due to the fixed positions of the proximal ends of the intermediate shaft and the inner shaft, it may not be possible to further reduce the balloon profile. The larger than desired balloon profile may result in elevated balloon retrieval forces when the delivery apparatus 100 is withdrawn from the patient's body.

Issues associated with larger than desired deflated balloons can be addressed by a delivery apparatus having an inflation hub assembly that permits adjustment of the overall length of the balloon, and therefore reduction of the profile of the balloon prior to removal of the delivery apparatus from the patient's body. FIG. 7 shows an interior region of a handle 102 and an inflation hub assembly 700 for a delivery apparatus, such as the delivery apparatus 100, according to one embodiment. The inflation hub assembly 700 in the illustrated embodiment comprises an inflation manifold 706 and a piston 708 slidably coupled to inflation manifold 706. The piston 708 can slide into and out of the inflation manifold 706 to adjust the length of the balloon 108, as further described below.

The inflation manifold 706 comprises a main body, which in the illustrated embodiment is in the form of a central shaft 710 defining a main lumen 712. The manifold 706 further includes an inflation port 714 defining an inflation lumen 716 that is in fluid communication with the main lumen 712 of the shaft 710. The central shaft 710 includes a distal end portion 718 and a proximal end portion 720. The distal end portion 718 defines a distal section 724 (or distal bore) of the main lumen 712. The proximal end portion 720 defines a proximal section 726 (or proximal bore) of the main lumen 712.

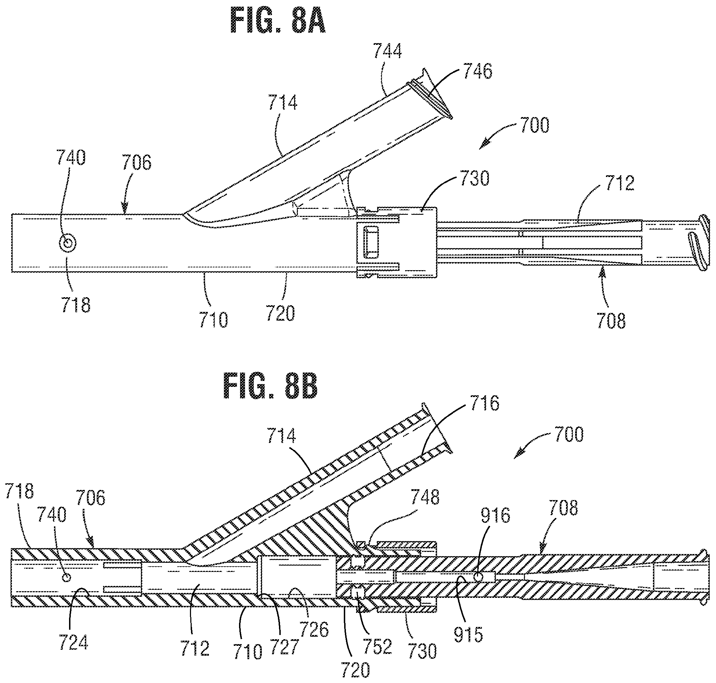

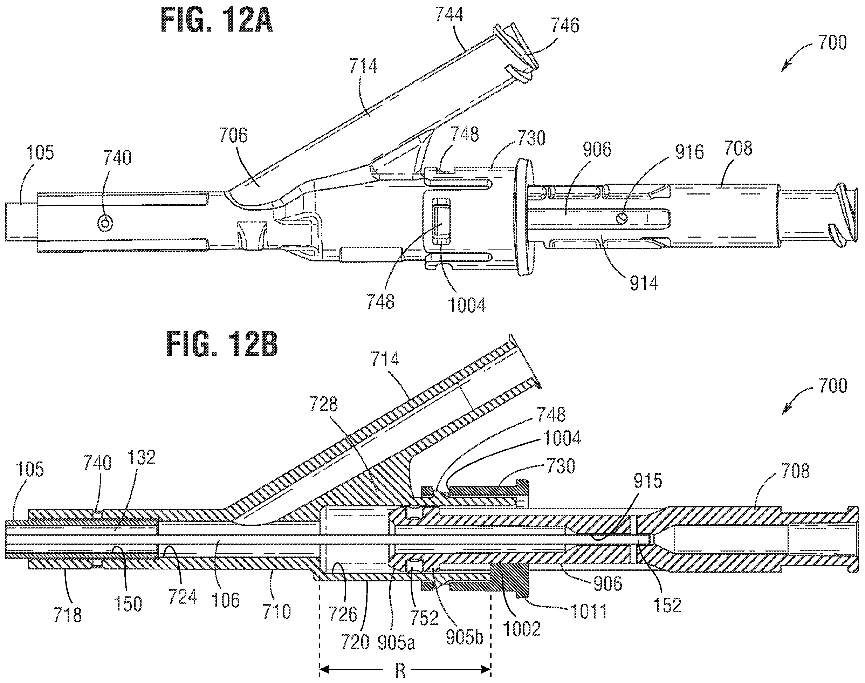

The proximal end portion 150 of the intermediate shaft 105 can be coupled to the inflation manifold 706 such that the intermediate shaft is at a fixed location and does not move axially relative to the inflation manifold 706. For example, as best shown in FIG. 12B, the proximal end portion 150 of the intermediate shaft 105 can extend into the distal bore 724 of the distal end portion 718 of the central shaft 710 and can be fixed at that position relative to the distal bore 724. The shaft distal end portion 718 may include a glue porthole 740 (also shown in FIGS. 8A and 8B) for receiving an adhesive for bonding the proximal end portion 150 of the intermediate shaft 105 with the inner surface of the distal bore 724. Still other techniques and mechanisms may be employed for securing the intermediate shaft 105 to the shaft 710, such as an interference fit, welding, chemical bonding, mechanical fasteners, snap fit features, and/or other fastening means. In some embodiments, the shaft 105 can be the outermost shaft of the delivery device (i.e., the shaft 104 can be omitted).

The proximal end portion 152 of the inner shaft 106 can be coupled to the piston 708 such that the inner shaft 106 is fixed axially relative to the piston 708 and axial movement of the piston in proximal and distal directions causes corresponding axial movement of the inner shaft 106. For example, as best shown in FIG. 12B, the proximal end portion 152 of the inner shaft can extend into a bore 914 of the piston 708 and can be fixed relative thereto. The piston 708 can include a glue porthole 916 (also shown in FIGS. 8A and 8B) for receiving an adhesive for bonding the proximal end portion 152 of the inner shaft 106 with the inner surface of the bore 914. Still other techniques and mechanisms may be employed for securing the inner shaft 106 to the piston 708, such as an interference fit, welding, chemical bonding, mechanical fasteners, snap fit features, and/or other fastening means.

Referring again to FIG. 7, the inflation hub assembly 700 can further include a cap member 730 that retains a distal end portion of the piston 708 within the bore 726, as further described below. In particular embodiments, the cap 730 can also maintain a rotational orientation of the piston 708 within the bore 726, as further described below.

The proximal bore 726 is sized to receive at least a distal end portion 902 (also referred to as a piston head) of the piston 708 and permit sliding movement of the piston within the proximal bore. As a result of the ability of the piston 708 to slide longitudinally within the bore 726 of the central shaft 706, the distal end of the piston 708 can be adjusted to any position in a range R (FIG. 12B) between a distal most position and a proximal most position. Correspondingly, the axial position of the inner shaft 106, which is coupled to the piston 708, moves upon movement of the piston 708. Since the distal end portion of the balloon 108 is coupled to the distal end portion of the inner shaft 106 (via the nose cone 110 in the illustrated embodiment), while a proximal end portion of the balloon is coupled to a proximal end portion of the intermediate shaft 105, changes in the position of the proximal end portion of the inner shaft (via adjustments to the piston position) result in corresponding changes in the length of the balloon.

As an example, when a balloon is deflated after delivery and deployment of a prosthetic device, such as a prosthetic heart valve, the balloon profile may be reduced by moving the piston 708 and the inner shaft 106 distally relative to the manifold 706 and the intermediate shaft 105. This moves the distal end portion 128 of the balloon 108 in a distal direction relative to the proximal end portion 126 of the balloon, which effectively increases the overall length of the balloon 108 and reduces its profile in a plane perpendicular to the longitudinal axis 124, thereby reducing balloon retrieval forces. Moreover, the increased length and smaller profile can promote refolding of the balloon as the inflation liquid is withdrawn from the balloon, further reducing balloon retrieval forces.

Referring again to FIG. 7, the manifold 706 can include a flange or gusset 728 to reinforce the connection between the inflation port 714 and the central shaft 710. In the illustrated embodiment, the inflation manifold 706 is disposed in the handle 102, except for a mouth portion 744 of the inflation port 714, which may extend out of the handle 102 of the delivery device for access by a user. The piston 708 in the illustrated embodiment can extend from the manifold 706 outwardly through a proximal opening in the handle where a proximal end portion of the piston 708 can be manipulated by a user.

In alternative embodiments, the inflation hub assembly 700, including the manifold 706 and the piston 708 can be located entirely outside of the handle 102. For example, the manifold 706 and the piston 708 can be located proximal to the handle 102, and the inner shaft 106 and the intermediate shaft 105 can extend completely through the handle and outwardly through a proximal opening in the handle for coupling to the piston and the manifold, respectively.

An inflation fluid may be delivered into the balloon 108 during balloon inflation via the inflation port 714, as previously described with respect to FIG. 6. In particular, fluid delivered into the inflation port 714 may flow through the lumen 716 into the main lumen 712 of the central shaft 710, and from thereon into an annular space 132 between the inner shaft 106 and the intermediate shaft 105 as previously described. Likewise, fluid may be withdrawn from the balloon during deflation via the inflation port. One or more grooves or external threads 746 (FIG. 8A) may be provided on the mouth portion 744 to facilitate coupling of the inflation port with a conduit (e.g., medical tubing) extending from a source of the inflation fluid, such as a syringe. The lumen 716 of the inflation port 714 can taper from the mouth portion 744 towards the gusset 728 at the junction with the central shaft 710.

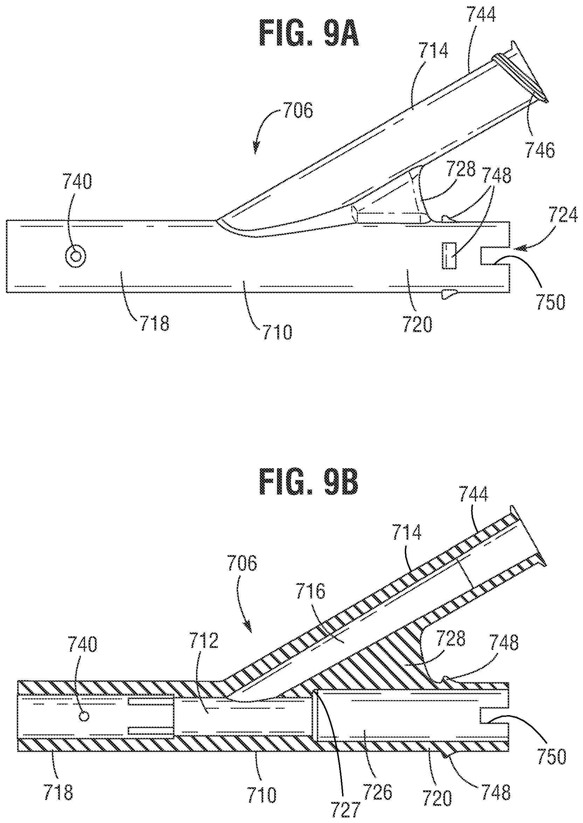

As best shown in FIGS. 9A and 9B, the proximal end portion 720 of the central shaft can include a plurality of projections 748 and at least one slot 750 to enable coupling of the cap member 730 with the proximal end portion 720 of the shaft 710. During assembly, the cap member 730 may be slid over the proximal end portion of the shaft 710 after piston 708 is inserted into the proximal bore 726.

The plurality of projections 748 extend radially outwardly from an outer surface of the proximal end portion of the shaft 710 and are circumferentially spaced from each other on the outer surface, distal to the slot 750. In the depicted example, the projections 748 are shaped as triangular prisms although the projections may have any other shape. The projections 748 are configured to act as snap fit retainers. Specifically, when the cap 730 is slid onto the proximal end portion 720 of the shaft 710, the projections 748 can extend into and form a snap fit connection corresponding windows (or openings) 1004 of the cap 730, thereby holding the cap 730 in place on the proximal end portion 720 of the shaft 710. As a result of the snap fit connection, axial movement of the cap 730 relative to the shaft 710 is prevented. Engagement of a rib 1002 of the cap 730 within a slot 750 of the shaft 710 can prevent rotation of the cap relative to the shaft, as further described below. As elaborated below, in certain embodiments, the cap 730 also holds the piston 708 in place so as to avoid rotation of the piston relative to the shaft 710.

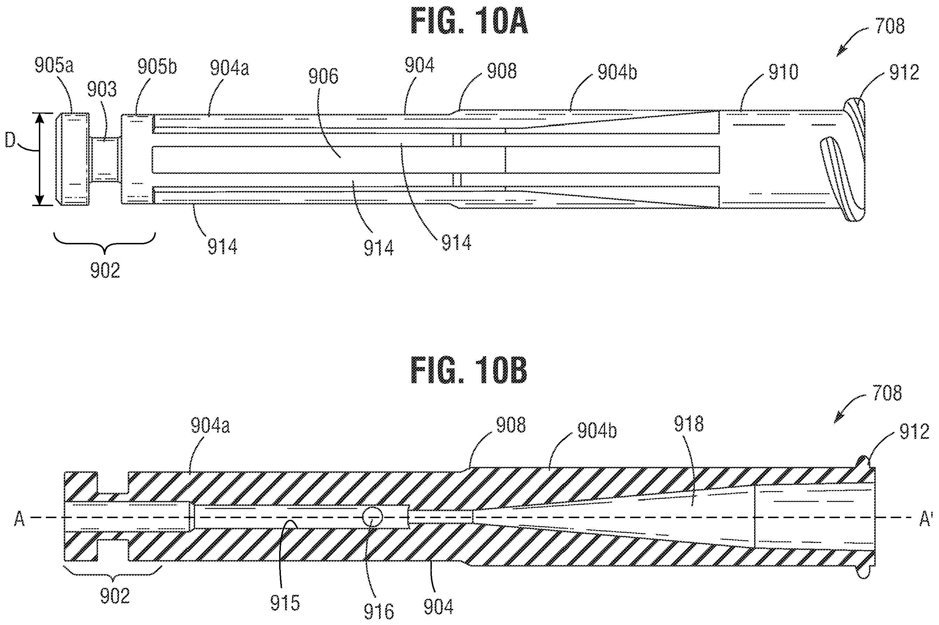

Referring to FIGS. 10A and 10B, the piston 708 has a piston head 902 and a piston stem 904 extending from the piston head 902. The piston head 902 includes a seal gland or annular groove 903 separating the piston head into a distal piston head region 905a and a proximal piston head region 905b. At least one seal 752 (see FIG. 8B) may be received within seal gland 903 on the piston head 902 to seal against the inner surface of the bore 726 to maintain pressurization of the inflation lumen. As non-limiting examples, the seal 752 may be an X-ring, an O-ring, a washer or the like. Although only one seal 752 is shown in the illustrated embodiment, in other embodiments, multiple seals 752 can be disposed in the groove 903. In alternative embodiments, multiple, axially-spaced grooves 903 can be provided along the piston head 902, with one or more seals disposed in each groove 903.

The piston stem 904 extends proximally from the proximal piston head region 905b. A plurality of ridges 914 protrude outwardly from the outer surface of the piston stem 904. The ridges are circumferentially distributed and each ridge extends longitudinally from the proximal head region 905b at least partially along a length of the piston stem. In one example, the ridges 914 are rectangular in shape and distributed uniformly across the circumference of the piston stem. As a result of the plurality of ridges 914, a series of grooves 906 are defined on the outer surface of the piston stem 904 between adjacent ridges. Rectangular ridges 914 result in the creation of rectangular grooves 906 interspersing the ridges. In other examples, the ridges 914 and the grooves 906 can have other cross-sectional profiles, such as triangular, semi-circular, etc. The grooves 906 also extend longitudinally at least partially along the length of the piston stem 904.

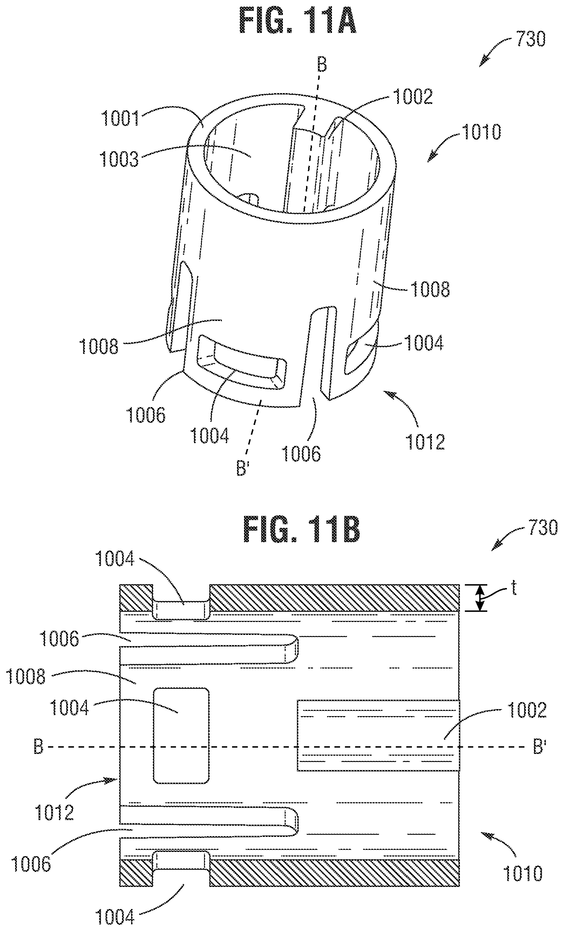

As elaborated below, a rotational orientation of the piston 708 within the shaft 710 of the manifold 706 can be maintained via the cap 730. During assembly, the piston head 902 is inserted into the proximal end portion 720 of the shaft 710, after which the cap 730 is placed over the proximal end portion 720, as previously described. This coupling results in the insertion of a rib 1002, provided on an inner surface of the cap 730 (see FIG. 11A), within one of the grooves 906 and the slot 750. Due to the engagement of the rib 1002 with the slot 750 in conjunction with the circumferential juxtaposition of the rib 1002 between two adjacent ridges 914, rotational motion of the piston is prevented. This prevents rotation of the piston once inserted inside the proximal end portion 720 of the shaft 710, which in turn prevents rotation of the inner shaft 106 and a guidewire (not shown) extending through the inner shaft 106. In alternative embodiments, the cap 730 can be fixed against rotation relative to the manifold 706, such as via the engagement of a rib 1002 with the slot 750, and the piston 708 can rotate relative to the cap 730 and the manifold 706. In such embodiments, the inner shaft 106 is fixed against axial movement relative to the piston 708, but the piston can rotate relative to the inner shaft 106. In this manner, rotation of the piston 708 in normal use does not produce rotation of the inner shaft 106 and a guidewire extending through the inner shaft.

The rib 1002 also limits proximal displacement of the piston inside the shaft 710, as elaborated below. When the piston 708 is moved proximally inside the manifold 706, the piston head moves towards the rib until they abut. Thereafter, the rib acts as a hard stop preventing further proximal movement of the piston in the manifold. In this way, a complete pulling out of the piston from the manifold can be averted.

When the piston 708 is moved distally inside the proximal bore portion 726 of the shaft 710, the distal piston head region 905a extends through the proximal bore portion 726 until it reaches an annular shoulder or edge 727 of the proximal bore portion 726. Beyond the shoulder 727 in the distal direction, the lumen 712 has a narrower width or diameter and therefore further distal movement of the piston within the lumen 712 of the shaft 710 is prevented. As described in detail below, movement of the piston 708 is effective to adjust the length of the balloon 108. The amount of movement of the piston in the distal direction (dimension R) can be selected to prevent excessive elongation of the balloon 108 which can result in tearing of the balloon.

The piston stem 904 can have a stepped diameter with a narrower distal stem region 904a that stepwise transitions to a wider proximal stem region 904b at a tapered junction 908. The grooves 906 in the illustrated embodiment do not necessarily extend the entire length of the piston stem 904. Thus, an outer surface of a proximal end portion 910 of the piston stem can be free of any grooves 906.

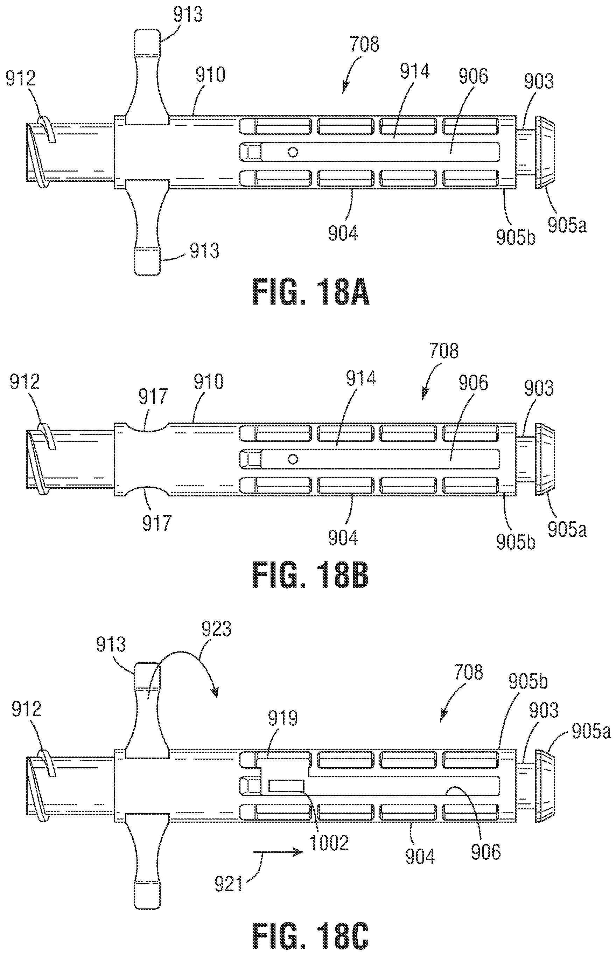

As shown in FIGS. 18A and 18B, the proximal end portion 910 of the piston stem 904 may have a finger grip feature to facilitate manual actuation of the piston by a user. In one example, as shown in FIG. 18A, the finger grip feature can comprise one or more protrusions or flanges 913 that extend radially outwardly from the piston stem. As shown in FIG. 18B, the finger grip feature can comprise one or more detents or notches 917 formed in the outer surface of the piston stem. A user may manually displace the piston 708 proximally and distally relative to the manifold 706 via the grip feature 913, 917. As shown in FIG. 7, at least the proximal end portion 910 of the piston stem 904, including the grip feature 913, may extend out of the handle 102 of the catheter, for access by the user. The proximal end portion 910 can also be formed with external threads 912 for connecting a luer fitting to the piston for injecting a liquid (e.g., saline) into the lumen of the piston and the inner shaft 106 for flushing those components.

Still other features may be provided on an external surface of the piston stem 904 to aid a user in manually actuating the piston to vary the length of the balloon. These include, as non-limiting examples, textural features (*e.g.,* the piston stem may be textured along the proximal end portion 910), and locking features (e.g., via the inclusion of twists, snaps, bayonet mounts, etc., on the proximal end portion 910 of the piston stem).

As best shown in FIG. 10B, the piston 708 includes a central bore or lumen 915 that extends through an entire length of the piston, along a central longitudinal axis A-A'. The lumen 915 can be narrower within the distal stem region 904a. Beyond the junction 908, within the proximal stem region 904b, the lumen can transition into a flared portion 918 which has a largest diameter at the proximal end of the piston stem 904. As discussed above, the lumen 915 is sized such that the proximal end portion 152 of the inner shaft 106 can be received within the lumen in the proximal stem region 904a, as shown in FIG. 12B.

Referring to FIGS. 11A-11B, the cap 730 includes a cylindrical wall 1001 of thickness "t" defining a hollow interior space 1003. The cap 730 has a diameter that is larger than the diameter of the proximal end portion 720 of the shaft 710 so as to allow the cap to be positioned over the proximal end portion 720. The cap 730 has a distal end portion 1012 and a proximal end portion 1010.

At the proximal end portion 1010 of the cap, the wall 1001 can be continuous while at the distal end portion 1012, the wall 1001 can be segmented. In some embodiments, as best shown in FIG. 12B, the proximal end portion 1010 of the cap 730 can be formed with a radially extending flange 1011 to enhance the circumferential strength of the cap. The distal end portion 1012 of the cap can include a plurality of rectangular notches or slots 1006 formed in the wall. The plurality of slots or notches 1006 are circumferentially arranged and can be uniformly distributed over the circumference of the cap at the distal end portion 1012. In other examples, the notches may be non-uniformly distributed.

Each notch 1006 extends through the wall, from the distal end portion 1012 towards the proximal end portion 1010. In the depicted example, the notches 1006 extend half a length of the cap, from the distal end to the proximal end. However, in other examples, the notches 1006 may extend more than half the distance, or less than half the distance. Each notch 1006 runs coaxial to a central axis B-B' of the cap 730.

As a result of the notches, the distal end portion 1012 of the cap is divided into a plurality of segments 1008. The number of segments 1008 is equal to the number of notches 1006. In the depicted example, the distal end portion 1012 includes four notches dividing the distal end of the cap into four segments. In other examples, a larger or smaller number of notches and segments may be provided. The segmented structure renders the distal end portion 1012 of the cap flexible, in contrast to the more rigid nature of the proximal end portion 1010 of the cap. Specifically, the segmented structure allows the cap 730 to be easily coupled to the proximal end portion 720 of the shaft 710, as further described below.

Each segment 1008 includes a window (or opening) 1004. The windows 1004 are shaped and sized to form a snap fit connection with projections 748 on the proximal end portion 720 of the shaft 710 when the cap 730 is coupled thereto. In the depicted example, the windows are rectangularly shaped to form a snap fit connection with the triangular prism shaped projections 748. Thus, as the cap 730 is placed over the proximal end portion 720 of the shaft 710 in a distal direction, the segments 1008 can flex outwardly as they move over the projections 748 until the projections 748 extend into corresponding windows 1004, at which point the segments 1008 can revert back to their non-deflected state, thereby retaining the cap 730 on the proximal end portion 720 of the shaft 710.

As further shown in FIGS. 11A and 11B, an axially extending rib 1002 can be provided on an inner surface of wall 1001. In one example, a single rib 1002 is provided. In other examples, additional ribs may be provided. The rib 1002 is coaxial to the central axis B-B' of the cap. The rib 1002 may be sized to be received within one of the grooves 906 on the piston stem 904. The rib 1002 extends along the proximal end portion 1010 of the cap towards the distal end portion. In one example, the rib extends half the length of the cap, as shown, although in other examples the rib 1002 can extend less than or greater than half the length of the cap

The dimensions of rib 1002 are selected to enable the rib to mate with one of the grooves 906 formed on the outer surface of piston 708 and the slot 750. Specifically, when the cap 730 is placed over the proximal end portion 720 of the shaft 710, the rib 1002 is inserted into, and mated with, with a groove 906 provided on the outer surface of the piston 708. The rib 1002 allows the piston 708 to be moved axially in the proximal and distal directions relative to the cap 730 and the manifold 706 and functions as a stop to limit proximal travel of the piston 708. Thus, when the piston 708 is moved in a proximal direction, such as via a pulling action on the piston stem 904 or pressurization during inflation of the balloon, the piston head 902 moves towards the rib 1002. When the piston head abuts the rib 1002, further proximal motion of the piston is prevented. Thus, the rib 1002 acts as a hard stop preventing the piston 708 from being pulled out of the shaft 710.

Moreover, the engagement of the rib 1002 with a groove 906 in the piston 708 prevents rotational movement of the piston 708 relative to the cap 730, which in turn is prevented from rotating relative to the manifold 706 via the connection between the rib 1002 and the slot 750. Since the inner shaft 106 is fixed relative to the piston 708, the engagement of the rib 1002 with a groove 906 and the rib 1002 with the slot 750 can prevent rotation of the shaft 106 and the piston 708 relative to the manifold 706 to prevent undesirable rotation of a guidewire extending through the inner shaft 106. In alternative embodiments, as previously described, the inner shaft 106 is only fixed against axial movement relative to the piston 708 and the piston 708 can be allowed to rotate relative to the cap 730, the manifold 706, the inner shaft 106, and the guidewire.

FIG. 13A shows an example balloon profile 1200 for a balloon catheter. The profile of FIG. 13A corresponds to a proximal position of the piston 708, shown in FIG. 13B. In one example, the piston position and balloon profile of FIGS. 13A and 13B may correspond to a profile provided when the balloon is deflated after valve delivery but in the absence of any distal piston movement (similar to a balloon profile after balloon deflation using the fixed design hub assembly of FIG. 6).

As discussed above, and as shown in FIG. 4, when a prosthetic valve 114 is initially crimped on the balloon 108 ready for implantation, the balloon 108 is deflated is tightly folded around the proximal and distal stops 120, 122, respectively. After the prosthetic valve 114 is deployed at the desired implantation site and the balloon is deflated, the balloon 108 may not revert back to its pre-deployment state in which it is tightly folded around the stops in a small profile. As a result, the balloon 108 can have a profile 1200 that is distended radially as shown in FIG. 13A. The larger or higher balloon profile can result in the need for relatively high balloon retrieval forces back through an introducer sheath, which can shear the balloon. For example, the retrieval force required with the higher balloon profile may be about 63.6 N when using a 14 Fr introducer sheath.

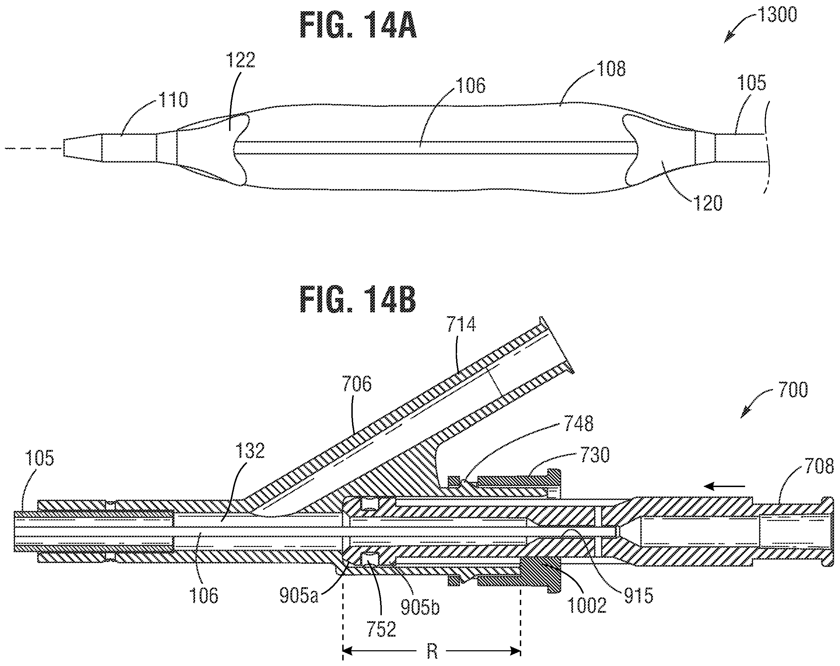

During or after deflating the balloon, a lower balloon profile, such as profile 1300 of FIG. 14A, can be achieved by moving the piston to its distal position, as shown in FIG. 14B. This results in the elongation of the balloon 108 between shoulders 120, 122 of the balloon shoulder assembly and a decrease in the overall profile of the balloon. Advantageously, this lowers the retrieval force required to retract the delivery device back through the introducer sheath and out of the patient's body. For example, the retrieval force required with the lower balloon profile can be about 18.2 N or less when using the same 14 Fr introducer sheath. In this way, by adjusting the position of the piston within the bore of the proximal hub component, a proximal end of the inner shaft can be varied to provide a desired degree of balloon profile elongation.

As discussed above, distal actuation of the inner shaft 106 may be desired during, or immediately after, a balloon deflation step (and after expansion of a prosthetic valve via the balloon). In one example, the distal motion of the piston may be provided manually, such as by a catheter operator. For example, the user may grip and push on a grip feature 912 on the piston stem to displace the piston manually a desired distance.

In another example, the distal movement of the piston may be vacuum actuated. The vacuum required for actuation may be provided from a vacuum source external to the delivery device. Alternatively, the vacuum required for vacuum actuation of the piston may be generated internal to the delivery device during fluid removal from the balloon. For example, as discussed above, a source of an inflation fluid (e.g., a syringe) may be fluidly connected to the inflation port 714, such as via medical tubing. When the balloon is filled with an inflation fluid to expand the prosthetic valve, a positive pressure is established inside the lumen 712 of the manifold 706. During balloon deflation, the inflation fluid is withdrawn from the annular space 132 and the manifold 706 back into the syringe. This creates a vacuum inside the lumen 712 of the manifold 706, which can draw the piston 708 distally within the proximal bore portion 726 without any manual forces applied to the piston 708. In particular embodiments, the vacuum is sufficient to move the piston to its distal position shown in FIG. 14B. Advantageously, as a result, distal piston displacement, and a resulting balloon elongation and a lower balloon profile, is achieved as fluid is removed to deflate the balloon.

In some embodiments, a larger vacuum can be achieved by increasing the diameter of the 708 piston and the diameter of the bore 726. In particular embodiments, the piston 708 has a diameter D (FIG. 10A) measured at the head portions 905a, 905b of the piston 708 in a range from 0.375 inch to 0.75 inch, or larger, which has been found to create sufficient vacuum to move the piston 708 from its proximal position (FIG. 13B) to its distal position (FIG. 14B) without a manual force applied to the piston. More desirably, a diameter D of least 0.5 inch has been found to create sufficient vacuum to begin moving the piston 708 toward its distal position immediately upon deflation of the balloon, which promotes refolding of the balloon as it is being deflated.

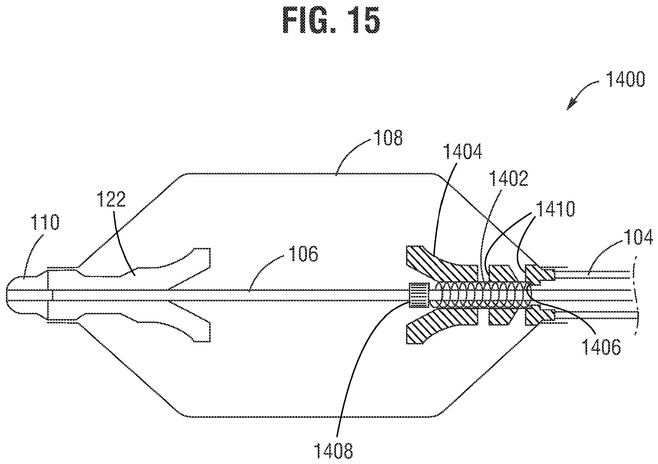

In still other embodiments, in lieu of or in addition to vacuum actuation, the hub assembly may include various features to allow for automatic distal actuation of the piston. As one example, as shown in delivery device 1400 of FIG. 15, a biasing element, such as the illustrated compression spring 1402, can be mounted within the balloon and configured to apply a biasing force that biases the inner shaft 106 to move toward the distal position. The spring 1402 can be mounted coaxially on the inner shaft 106 at least partially within an inner bore of a proximal shoulder 1404. A proximal end of the spring 1402 can abut an inner surface 1406 of the shoulder 1404 and a distal end of the spring 1402 can abut a stop member or collar 1408 that is fixed to the inner shaft 106. A distal end of the balloon 108 is fixed to a distal shoulder 122 and/or a nosecone 110 and a proximal end of the balloon is fixed to the proximal shoulder, as previously described. In this manner, the biasing force of the spring 1402 moves the inner shaft 106 and the distal end of the balloon distally relative to the outer shaft 104, the handle 102, and the proximal end of the balloon to elongate the balloon.

It should be noted that the delivery device 1400 can include any of the features described above in FIGS. 3-5 and 7-14B, including the hub assembly 700 with the manifold 706, the piston 708, and the cap 730. Unlike the embodiment shown in FIG. 4, in the embodiment of FIG. 15 the intermediate shaft 705 is omitted. Thus, the proximal shoulder 1404 can be mounted to a distal end portion of the outer shaft 104 and a flow path for inflation fluid is defined between the outer shaft 104 and the inner shaft 106. The fluid path can be in fluid communication with the inflation lumen 716 of the inflation manifold 706 within the handle 102. At the distal end of the outer shaft 104, the inflation fluid through the proximal shoulder 1404 and the spring 1402 into the balloon to inflate the balloon. The proximal shoulder 1404 can further include inflation ports or openings 1410 that allow inflation fluid within the proximal shoulder to flow radially outwardly into the interior of the balloon. In alternative embodiments, the spring 1402, the proximal shoulder 1404, and the stop member 1408 can be implemented in a delivery device having the intermediate shaft 105, such as shown in FIG. 4.

The biasing force of the spring 1402 may be selected to be low enough to allow the inner shaft 106 to be manually moved to the proximal position (as shown in FIGS. 13A and 13B) for folding the balloon 108 around the shoulders 122, 1402 and for crimping a prosthetic valve (e.g., valve 10, 50) on the balloon between the shoulders. The compression force of the prosthetic valve against the balloon and the inner shaft can maintain the inner shaft 106 in the proximal position (and prevent elongation of the balloon) against the bias of the spring 1402 as the prosthetic valve is advanced to the desired implantation site. As the balloon is inflated at the implantation site to deploy the prosthetic valve, the compression force of the prosthetic valve is released and the spring 1402 automatically moves the inner shaft 106 to the distal position (as shown in FIGS. 14A and 14B) to elongate the balloon. Thereafter, the balloon can be deflated and withdrawn from the patient's body.

In some embodiments, the inflated balloon 108 can retain the inner shaft 106 in the proximal position (preventing balloon elongation) against the biasing force of the spring, but permits distal movement of the inner shaft 106 as the balloon is deflated under the force of the spring. Thus, in this manner, the balloon elongates as the balloon is being deflated.

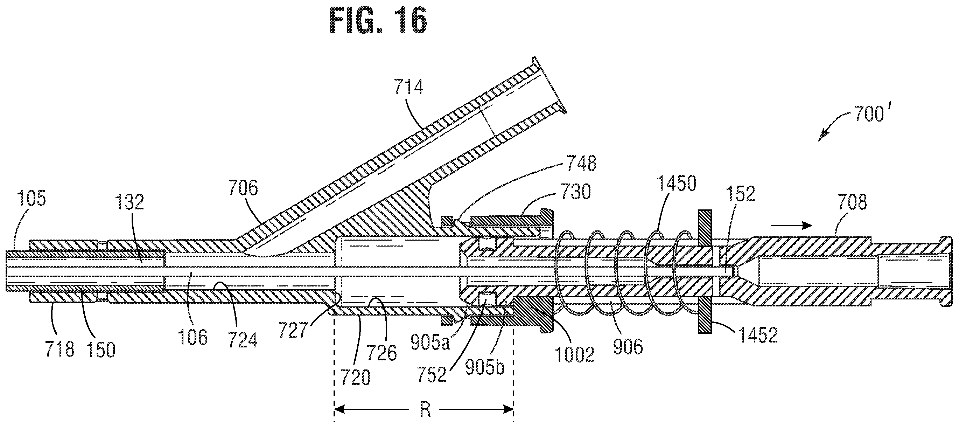

In alternative embodiments, a biasing element can be provided at the proximal end of the delivery device to bias the inner shaft 106 to the distal position. As shown in FIG. 16, a hub assembly 700'can be similar to hub assembly 700 except for a tension spring 1450 mounted on the piston 708. A distal end of the spring 1450 can be connected to the cap member 730 and a proximal end of the spring 1450 can be connected to a rib 1452 (or other another surface) of the piston 708. The spring 1450 is configured to apply a biasing force to the piston 708 and the inner shaft 106 to move the inner shaft to the distal position (as shown in FIGS. 14A and 14B) to elongate the balloon under the force of the spring. Similar to the spring 1402, the force of the spring 1450 can be selected to allow the inner shaft 106 to be moved to the proximal position for folding the balloon 108 and crimping the prosthetic valve on the balloon. The crimped prosthetic valve can retain the inner shaft 106 in the proximal position until the balloon is inflated, at which point the inner shaft 106 can move to the distal position to elongate the balloon under the biasing force of the spring. In other embodiments, the inflated balloon 108 can retain the inner shaft 106 in the proximal position (preventing balloon elongation) against the biasing force of the spring 1450, but permits distal movement of the inner shaft 106 as the balloon is deflated under the force of the spring. Thus, in this manner, the balloon elongates as the balloon is being deflated

In some examples, the piston displacement can be actuated passively and/or actively. For example, during balloon deflation, the piston may be distally displaced to a first distal position via passive forces (such as from a biasing device (e.g., a spring 1402 or 1450) or vacuum forces), thereby providing a first amount of balloon elongation. If further balloon elongation and a lower balloon profile is desired, a user can then further displace the piston distally, such as to a second distal position, via active manual adjustments. In one example, this may enable maximal elongation of the balloon.

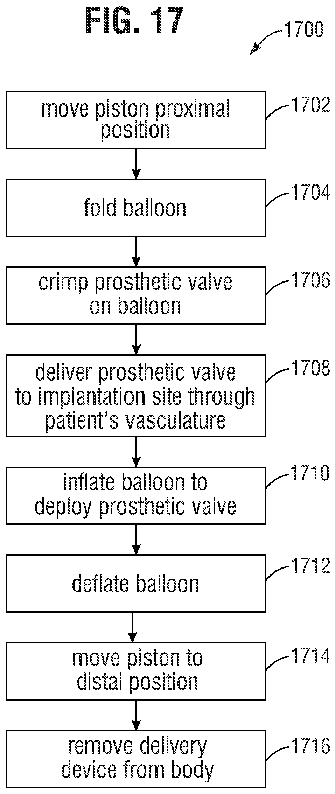

FIG. 17 shows an example method 1700 of operating an inflation hub assembly to adjust a balloon profile of a balloon catheter during delivery of a prosthetic valve and subsequently during balloon retrieval.

At 1702, the method includes actuating or moving a piston of the hub assembly to a proximal position while the balloon is deflated prior to crimping a prosthetic valve 114 on the balloon 108. In one example, the proximal position includes the piston head 902 abutting the inner rib 1002 of the cap 730 (e.g., as shown in FIG. 13B). Actuating or moving the piston to the proximal position includes providing the balloon with a larger (less elongated) balloon profile.

In some implementations, the initial proximal position of the piston 708 can be a location where the piston head 902 is spaced slightly distally from the rib 1002 such that the piston head does not abut the rib 1002. This allows the piston 708 to move proximally at certain times during the implantation procedure, as further discussed below.

In this state, as shown at 1704, the balloon 108 may be folded around the proximal and distal stops 120, 122 and the prosthetic valve 114 can be radially crimped around the balloon 108, as shown at 1706. In certain embodiments, the balloon 108 can be pre-folded during manufacturing and delivered to the end user in a folded state. The end user (e.g., a physician) can remove the delivery apparatus from its sterile package and then crimp the prosthetic valve on the folded, deflated balloon. In other embodiments, the prosthetic valve 114 can be crimped onto the balloon 108 at the manufacturing site and shipped to the end user with prosthetic valve pre-crimped on the balloon.

Once the prosthetic valve 114 is crimped on the balloon, the physician can insert the prosthetic valve and the delivery catheter into a patient's vasculature and advance the prosthetic valve to the desired implantation site, as shown at 1708. For example, when replacing a native aortic valve, the prosthetic valve and the delivery apparatus can be inserted into the aorta via an incision in a femoral artery, advanced through the descending aorta, the aortic arch, and the ascending aorta until the prosthetic valve 114 is positioned within the native aortic valve. Prior to crossing the native aortic valve, if the initial position of the piston head 902 is at a location spaced from the rib 1002, the user can pull the piston proximally to bring the shoulders 120, 122 closer to the adjacent ends of the prosthetic valve, thereby minimizing or closing any gaps between the ends of the prosthetic valve and the shoulders 120, 122, which can facilitate crossing the native valve.

At the desired implantation site, the prosthetic valve can be expanded by inflating the balloon 108, as shown at 1710. If the initial position of the piston head 902 is at a location spaced from the rib 1002, positive pressure within the manifold 706 can move the piston proximally until it contacts the rib 1002, which promotes full inflation of the balloon.

After deploying the prosthetic valve, the balloon 108 is then deflated by withdrawing the inflation fluid from the balloon, as shown at 1712.

At 1714, the method includes actuating the piston of the hub assembly to a distal position while or after the balloon is deflated in order to elongate the balloon. Actuating the piston to the distal position may include a user (e.g., a physician or technician) actuating the piston manually, and/or actively, via a biasing element and/or vacuum forces inside the manifold 706 as previously described. At 1716, the delivery device can be removed from the body.

In some embodiments, the piston 708 and/or the cap 730 can have a locking feature that can be selectively actuated to retain the piston in the distal position during retrieval of the delivery device from the patient's body. In one implementation, as shown in FIG. 18C, the piston stem 904 can include a notch 919 that is in communication with a groove 906 in which the rib 1002 of the cap 730 is disposed. As the piston 708 is moved distally (in the direction of arrow 921) to elongate the balloon, the groove 906 moves relative to the rib 1002 until the rib 1002 is located at the distal end of the groove adjacent the notch 919, as depicted in FIG. 18C. The piston 708 can then be rotated (in the direction of arrow 923) relative to the cap 730 to position the rib 1002 within the notch 919. Once the rib 1002 is within the notch 919, the notch 919 prevents axial movement of the piston 708 relative to the cap 730 (and the manifold 706), thereby fixing the position of the inner shaft 152 and retaining the balloon in the elongated state as the delivery device is withdrawn from the body.

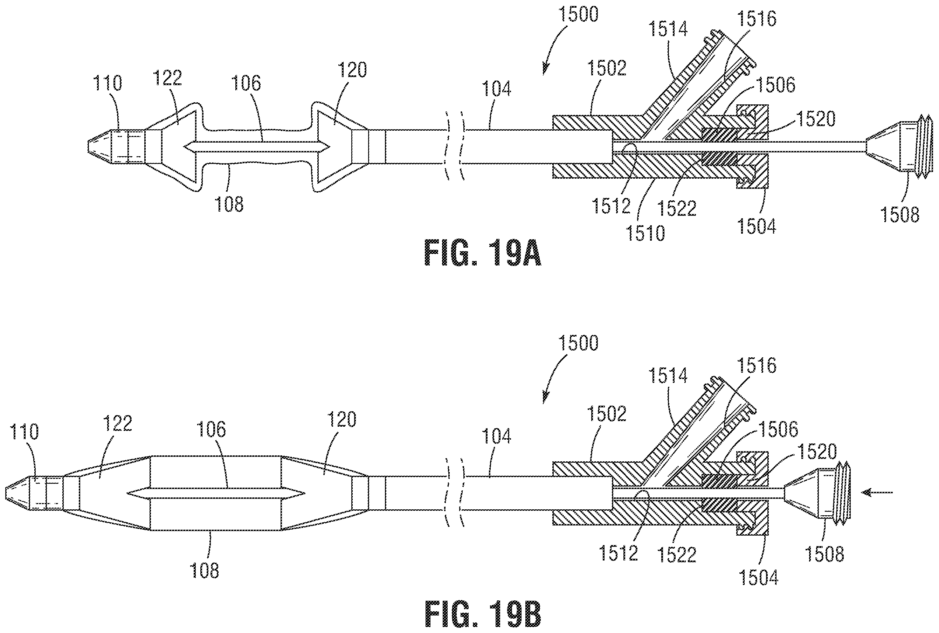

FIGS. 19A and 19B show a delivery device 1500, according to another embodiment. The delivery device 1500 includes many of the same features as the delivery device described above in FIGS. 3-5 and 7-14B. Thus, like components in FIGS. 19A and 19B and FIGS. 3-5 and 7-14Bare given the same reference numerals and are not described in detail here.

The delivery device 1500 in the illustrated embodiment includes an inflation hub assembly comprising a manifold 1502, a cap 1504, a sealing member 1506, and a hub 1508. The manifold 1502 includes a main body in the form of a central shaft 1510 defining a main lumen 1512. The manifold further includes an inflation port 1514 defining an inflation lumen 1516 that is in communication with the main lumen 1512. In some embodiments, the manifold 1502 can be disposed in a handle (e.g., handle 102) with the inflation port 1514, the proximal end of the shaft 1510, the cap 1504, the proximal end of the shaft 106, and the hub 1508 positioned outside of the handle for access by a user, similar to the configuration shown in FIG. 7. In other embodiments, the manifold 1502 can function as a handle.

A proximal end portion of the outer shaft 104 can extend into an enlarged distal section of the main lumen 1512 and can be fixed relative thereto, such as via a press fit, welding, an adhesive, etc. A proximal end portion of the inner shaft 106 can extend through the main lumen 1512, the sealing member 1506, and the cap 1504 and can be fixed to the hub 1508 proximal to the cap 1504. Similar to FIG. 15, the intermediate shaft 105 is omitted and the proximal shoulder 120 can be mounted to the outer shaft 104 and an inflation path can be defined between the inner shaft 106 and the outer shaft 104. The main lumen 1512 can be in fluid communication with the fluid pathway to deliver an inflation fluid from the inflation port 1514 to the balloon 108.

The inner shaft 106 can be moved proximally and distally relative to the outer shaft 104 and the manifold 1502 to adjust the length of the balloon 108, as previously described. The cap 1504 is mounted on a proximal end portion of the shaft 1510 and is configured to selectively actuate the sealing member 1506 and resist movement of the inner shaft 106 when desired during an implantation procedure. **In** the illustrated embodiment, the cap 1504 is threadably coupled to the proximal end portion of the shaft 1510; for example, the cap 1504 can have internal threads that engage external threads on the proximal end portion of the shaft 1510 as shown. Alternatively, the cap have external threads that engage internal threads of the proximal end portion of the shaft 1510. **In** either case, rotation of the cap 1504 relative to the shaft 1510 causes the cap to move axially relative to the shaft (proximally or distally, depending on the direction of the rotation).

The cap 1504 has an inner portion 1520 (which can be a cylindrically shaped wall) that bears against the sealing member 1506. The sealing member 1506 is disposed in a proximal section of the main lumen 1512 and is captured between the inner portion 1520 of the cap and a radially extending wall or shoulder 1522 of the main lumen. The sealing member 1506 can be cylindrical in shape as shown and can be formed from an elastomeric material that permits linear and radial compression of the sealing member. The sealing member 1506 can be made from any of various elastomers, such as silicone rubber.

To fix the axial position of the inner shaft 106 relative to the outer shaft 104, the cap 1504 can be rotated in a first direction (e.g., clockwise) to move the cap distally on the shaft 1510, which causes the inner portion 1520 to bear against the sealing member 1506. This causes the sealing member to be compressed linearly between the inner portion 1520 and the shoulder 1522 and radially inwardly against the inner shaft 106. The force of the sealing member 1506 against the outer surface of the inner shaft 106 retains the inner shaft 106 against axial movement relative to the outer shaft 104, and therefore prevents adjustment of the balloon length. **In** this manner, the cap functions as a retaining mechanism to selectively retain the inner shaft 106 against axial movement relative to the outer shaft 106.

Rotating the cap 1504 in a second direction, opposite the first direction, (e.g., counterclockwise) moves the cap proximally on the shaft 1510 away from the sealing member 1506 and allows the sealing member to return to its undeformed state. In the undeformed state, the inner shaft 106 can be easily moved relative to the outer shaft 104 to adjust the length of the balloon 108.

FIG. 19A shows the sealing member 1506 in a locked (deformed) state retaining the inner shaft 106 in a proximal position, in which the balloon can be folded around the shoulders 120, 122 and a prosthetic valve (not shown) can be crimped onto the balloon as previously described. In this state, the delivery device and the prosthetic valve can be inserted into a patient's vasculature and advanced to a desired implantation site (e.g., the native aortic valve). Prior to inflating the balloon 108 and deploying the prosthetic valve, the cap 1504 can be loosened to allow the sealing member to return to its undeformed state and allow movement of the inner shaft during balloon inflation.

After expanding the prosthetic valve or during balloon inflation, the inner shaft 106 can be moved to a distal position (FIG. 19B) to elongate the balloon. Thereafter, the cap 1504 can be tightened to compress the sealing member 1506 and retain the position of the inner shaft 106 during removal of the delivery device from the patient's body, as shown in FIG. 19B.

In alternative embodiments, the cap 1504 can be configured for translational movement relative to the shaft 1510, such as by manually pushing and pulling the cap relative to the shaft 1510, instead of rotating the cap. Pushing the cap 1504 distally deforms the sealing member and retains the inner shaft while pulling the cap 1504 proximally allows the sealing member to revert to its undeformed state and permit movement of the inner shaft.

In alternative embodiments, the cap 1504 and the sealing member 1506 can be implemented in the hub assembly 700 to selectively retain movement of the piston 708. For example, the cap 730 can be configured to be rotatable relative to the shaft 710 so as to compress a sealing member 1506 (disposed in lumen 726) against the piston 708 and retain the piston 708 (and the inner shaft 106) against axial movement.

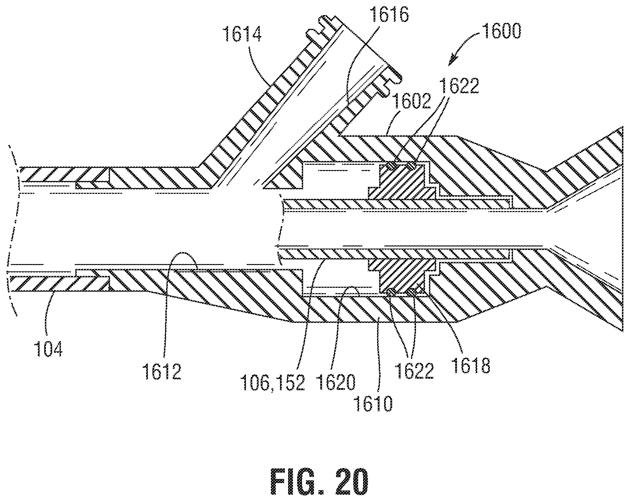

FIG. 20 shows an inflation hub assembly 1600, according to another embodiment, which can be implemented in any of the delivery device disclosed herein. The hub assembly 1600 comprises an inflation manifold 1602. The manifold 1602 includes a main body in the form of a central shaft 1610 defining a main lumen 1612. The manifold further includes an inflation port 1614 defining an inflation lumen 1616 that is in communication with the main lumen 1612. In some embodiments, the manifold 1602 can be disposed in a handle (e.g., handle 102) with the inflation port 1614 and the proximal end of the shaft 1610 positioned outside of the handle for access by a user, similar to the configuration shown in FIG. 7. In other embodiments, the manifold 1602 can function as a handle.

The proximal end portion 152 of the inner shaft 106 can be fixed, such as with an adhesive, welding, or press fit, to a moveable piston 1618 disposed in a proximal section 1620 of the main lumen 1612. A proximal end portion of the shaft 104 can be fixed, such as with an adhesive, welding, or press fit, to a distal end portion of the shaft 1610 of the manifold 1602. One or more sealing members 1622 can be disposed around the piston 1618, which create a fluid tight seal with the inner surface of the proximal section 1620 of the lumen 1612. The sealing members 1622 can be X-rings, O-rings, or other suitable configurations. The distal end portions of the shafts 104, 106 can be coupled to respective end portions of a balloon 108, as previously described and shown in FIGS. 19A-19B, for example. The piston 1618 can travel proximally and distally within the proximal section 1620 of the lumen 1612, producing corresponding movement of the shaft 106 relative to the shaft 104 and correspondingly changing the length of the balloon 108.

In the illustrated embodiment, the proximal end portion 152 of the inner shaft 106 and the piston are not accessible for manipulation by a user. For example, as shown in FIG. 20, the proximal end portion 152 of the inner shaft 106 terminates at a location within the manifold 1602 and the piston 1618 is located entirely within the manifold 1602. In some embodiments, the manifold 1602 can be located within a handle (e.g., handle 102) and the proximal end portion 152 of the inner shaft 106 can be extend outside of the manifold 1602 but terminates at a location within the handle such that it is not accessible to a user during normal use.

Thus, in this embodiment, movement of the piston 1618 (and therefore adjustment of the balloon length) can be entirely passive, i.e., movement of the piston occurs through normal use of the delivery device and does not require manual forces applied by a user. For example, during balloon inflation, positive pressure in the lumen 1612 can causes the piston 1618 to move proximally to promote full balloon inflation. During balloon deflation, negative pressure (vacuum) within the lumen 1612 can cause the piston 1618 to move distally, thereby elongating the balloon 108. Additionally, withdrawing the delivery device through an introducer sheath can create a frictional force against the balloon 108, which can pull the distal end portion of the balloon distally relative to the proximal end portion of the balloon, which further elongates the balloon and/or helps maintain the elongated state of the balloon as it is being withdrawn from the introducer sheath.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope of these claims.

## Claims

1. A delivery apparatus (100; 1400) for an implantable prosthetic device (10; 50; 114), the delivery apparatus (100; 1400) comprising:
a handle (102);
a first shaft (106) extending distally from the handle (102);
a second shaft (104; 105) extending distally from the handle (102), wherein the first shaft (106) extends through the second shaft (104; 105) and is movable axially relative to the second shaft (104; 105);
an inflatable balloon (108) having a proximal end portion (126) and a distal end portion (128), wherein the proximal end portion (126) of the balloon (108) is coupled to a distal end portion (152) of the second shaft (104; 105) and the distal end portion (128) of the balloon (108) is coupled to a distal end portion (112) of the first shaft (106); and
an inflation hub assembly (700; 700'; 1600) comprising an inflation manifold (706; 1602) and a piston (708; 1618), wherein the inflation manifold (706; 1602) comprises a main body defining a main lumen (712; 1612) extending therethrough and
an inflation port defining an inflation port lumen in fluid communication with the main lumen (712; 1612), wherein the piston (708; 1618) extends into the main lumen (712; 1612) and is slidable relative to the inflation manifold (706; 1602);
wherein a proximal end portion of the first shaft (106) is coupled to the piston (708; 1618) and a proximal end portion of the second shaft (104; 105) is coupled to the main body of the inflation manifold (706; 1602) or the handle (102);
wherein the piston (708; 1618) is moveable relative to the inflation manifold (706; 1602) in proximal and distal directions such that distal movement of the piston (708; 1618) produces distal movement of the first shaft (106) relative to the second shaft (104; 105), which increases the length of the balloon (108), and such that proximal movement of the piston (708; 1618) produces proximal movement of the first shaft (106) relative to the second shaft (104; 105), which decreases the length of the balloon (108).

2. The delivery apparatus (100; 1400) of claim 1, wherein the piston (708; 1618) comprises a piston head (902) and a piston stem (904), wherein the piston head (902) comprises an annular groove (903) and an annular seal member (726) is disposed in the annular groove (903), wherein the seal member (726) establishes a seal against an inner surface of the main lumen (712; 1612) of the inflation manifold (706; 1602)

3. The delivery apparatus (100; 1400) of any preceding claim, wherein the proximal end portion of the first shaft (106) extends into and is secured within a lumen (915) of the piston (708; 1618).

4. The delivery apparatus (100; 1400) of any preceding claim, wherein the main lumen (712; 1612) of the inflation manifold (706; 1602) includes a radially projecting inner wall that is shaped to limit distal movement of the piston (708; 1618) within the main lumen (712; 1612).

5. The delivery apparatus (100; 1400) of any preceding claim, further comprising a cap member (730) disposed on a proximal end portion of the main body (602) of the inflation manifold (706; 1602), wherein the cap member (730) comprises a projection (748) positioned to limit proximal movement of the piston (708; 1618) within the main lumen (712; 1612), wherein preferably the projection (748) extends radially into the main lumen (712; 1612) of the inflation manifold (706; 1602).

6. The delivery apparatus (100; 1400) of claim 5, wherein the projection (748) comprises a rib (1002) that extends into an axially extending groove (903) formed on an outer surface of the piston (708; 1618), wherein the rib (1002) prevents rotation of the piston (708; 1618) relative to the cap member (730).

7. The delivery apparatus (100; 1400) of claim 5 or 6, wherein the cap member (730) forms a snap fit connection with the proximal end portion of the main body (602) of the inflation manifold (706; 1602).

8. The delivery apparatus (100; 1400) of claim 7, wherein the cap member (730) comprises a plurality of openings (1004) and the proximal end portion of the main body (602) of the inflation manifold (706; 1602) comprises a plurality of projections (748) sized to extend into the openings (1004) to form the snap fit connection.

9. The delivery apparatus (100; 1400) of any of claims 1 to 4, further comprising a cap member (730) disposed on a proximal end portion of the main body (602) of the inflation manifold (706; 1602), wherein the cap member (730) is configured to permit axial movement of the piston (708; 1618) and the first shaft (106) relative to the cap member (730) in the proximal and distal directions and resist rotational movement of the piston (708; 1618) and the first shaft (106) relative to the cap member (730).

10. The delivery apparatus (100; 1400) of any preceding claim, wherein the main lumen (712; 1612) is in fluid communication with a fluid pathway between the first and second shafts (104; 105, 106), which is in turn is in fluid communication with the balloon (108) such that an inflation fluid introduced into the inflation port lumen can flow through the main lumen (712; 1612), the fluid pathway, and into the balloon (108) to inflate the balloon (108).

11. The delivery apparatus (100; 1400) of claim 10, wherein the inflation hub assembly (700; 700'; 1600) is configured such that withdrawing the inflation fluid from the balloon (108) via the inflation port is effective to establish a vacuum in the inflation manifold (706; 1602) that moves the piston (708; 1618) and the first shaft (106) in the distal direction.

12. The delivery apparatus (100; 1400) of any preceding claim, further comprising a biasing member (1402; 1450) configured to bias the first shaft (106) to move in the distal direction relative to the second shaft (104; 105).

13. The delivery apparatus of claim 12, wherein the biasing member (1402; 1450) comprises a spring (1402; 1450).

14. The delivery apparatus (100; 1400) of claim 13, wherein the spring (1402; 1450) is disposed around the first shaft (106) within the balloon (108), or around the piston (708; 1618).

15. The delivery apparatus (100; 1400) of any preceding claim, wherein the main body (602) of the inflation manifold (706; 1602) is disposed in the handle (102).

## Patentansprüche

1. Zuführvorrichtung (100; 1400) für eine implantierbare Prothesenvorrichtung (10; 50; 114), wobei die Zuführvorrichtung (100; 1400) umfasst:
einen Griff (102);
einen ersten Schaft (106), der sich distal von dem Griff (102) erstreckt;
einen zweiten Schaft (104; 105), der sich distal von dem Griff (102) erstreckt, wobei der erste Schaft (106) sich durch den zweiten Schaft (104; 105) erstreckt und axial relativ zu dem zweiten Schaft (104; 105) bewegbar ist;
einen aufpumpbaren Ballon (108) mit einem proximalen Endabschnitt (126) und einem distalen Endabschnitt (128), wobei der proximale Endabschnitt (126) des Ballons (108) mit einem distalen Endabschnitt (152) des zweiten Schafts (104; 105) gekoppelt ist und der distale Endabschnitt (128) des Ballons (108) mit einem distalen Endabschnitt (112) des ersten Schafts (106) gekoppelt ist; und
eine Aufpumpanschlussanordnung (700; 700'; 1600), die einen Aufpumpverteiler (706; 1602) und einen Kolben (708; 1618) umfasst, wobei der Aufpumpverteiler (706; 1602) einen Hauptkörper umfasst, der ein sich dadurch erstreckendes Hauptlumen (712; 1612) definiert, und
einen Aufpumpanschluss, der ein Aufpumpanschlusslumen in Fluidverbindung mit dem Hauptlumen (712; 1612) definiert, wobei der Kolben (708; 1618) sich in das Hauptlumen (712; 1612) erstreckt und relativ zu dem Aufpumpverteiler (706; 1602) verschiebbar ist;
wobei ein proximaler Endabschnitt des ersten Schafts (106) mit dem Kolben (708; 1618) gekoppelt ist und ein proximaler Endabschnitt des zweiten Schafts (104; 105) mit dem Hauptkörper des Aufpumpverteilers (706; 1602) oder dem Griff (102) gekoppelt ist;
wobei der Kolben (708; 1618) relativ zu dem Aufpumpverteiler (706; 1602) in proximaler und distaler Richtung bewegbar ist, sodass eine distale Bewegung des Kolbens (708; 1618) eine distale Bewegung des ersten Schafts (106) relativ zu dem zweiten Schaft (104; 105) erzeugt, was die Länge des Ballons (108) vergrößert, und sodass eine proximale Bewegung des Kolbens (708; 1618) eine proximale Bewegung des ersten Schafts (106) relativ zu dem zweiten Schaft (104; 105) erzeugt, was die Länge des Ballons (108) verringert.

2. Zuführvorrichtung (100; 1400) gemäß Anspruch 1, wobei der Kolben (708; 1618) einen Kolbenkopf (902) und einen Kolbenschaft (904) umfasst, wobei der Kolbenkopf (902) eine ringförmige Nut (903) umfasst und ein ringförmiges Dichtelement (726) in der ringförmigen Nut (903) angeordnet ist, wobei das Dichtelement (726) eine Abdichtung gegen eine innere Oberfläche des Hauptlumens (712; 1612) des Aufpumpverteilers (706; 1602) schafft.

3. Zuführvorrichtung (100; 1400) gemäß einem der vorhergehenden Ansprüche, wobei der proximale Endabschnitt des ersten Schafts (106) sich in ein Lumen (915) des Kolbens (708; 1618) erstreckt und darin gesichert ist.

4. Zuführvorrichtung (100; 1400) gemäß einem der vorhergehenden Ansprüche, wobei das Hauptlumen (712; 1612) des Aufpumpverteilers (706; 1602) eine radial vorstehende Innenwand umfasst, die dazu ausgeformt ist, eine distale Bewegung des Kolbens (708; 1618) innerhalb des Hauptlumens (712; 1612) zu begrenzen.

5. Zuführvorrichtung (100; 1400) gemäß einem der vorhergehenden Ansprüche, ferner umfassend ein Kappenelement (730), das an einem proximalen Endabschnitt des Hauptkörpers (602) des Aufpumpverteilers (706; 1602) angeordnet ist, wobei das Kappenelement (730) einen Vorsprung (748) umfasst, der dazu positioniert ist, eine proximale Bewegung des Kolbens (708; 1618) innerhalb des Hauptlumens (712; 1612) zu begrenzen, wobei vorzugsweise der Vorsprung (748) sich radial in das Hauptlumen (712; 1612) des Aufpumpverteilers (706; 1602) erstreckt.

6. Zuführvorrichtung (100; 1400) gemäß Anspruch 5, wobei der Vorsprung (748) eine Rippe (1002) umfasst, die sich in eine axial verlaufende Nut (903) erstreckt, die an einer äußeren Oberfläche des Kolbens (708; 1618) ausgebildet ist, wobei die Rippe (1002) eine Rotation des Kolbens (708; 1618) relativ zu dem Kappenelement (730) verhindert.

7. Zuführvorrichtung (100; 1400) gemäß Anspruch 5 oder 6, wobei das Kappenelement (730) eine Schnappverbindung mit dem proximalen Endabschnitt des Hauptkörpers (602) des Aufpumpverteilers (706; 1602) ausbildet.

8. Zuführvorrichtung (100; 1400) gemäß Anspruch 7, wobei das Kappenelement (730) mehrere Öffnungen (1004) umfasst und der proximale Endabschnitt des Hauptkörpers (602) des Aufpumpverteilers (706; 1602) mehrere Vorsprünge (748) umfasst, die so bemessen sind, dass sie sich in die Öffnungen (1004) erstrecken, um die Schnappverbindung auszubilden.

9. Zuführvorrichtung (100; 1400) gemäß einem der Ansprüche 1 bis 4, ferner umfassend ein Kappenelement (730), das an einem proximalen Endabschnitt des Hauptkörpers (602) des Aufpumpverteilers (706; 1602) angeordnet ist, wobei das Kappenelement (730) so konfiguriert ist, dass es eine axiale Bewegung des Kolbens (708; 1618) und des ersten Schafts (106) relativ zum Kappenelement (730) in der proximalen und der distalen Richtung erlaubt und sich einer Drehbewegung des Kolbens (708; 1618) und des ersten Schafts (106) relativ zum Kappenelement (730) widersetzt.

10. Zuführvorrichtung (100; 1400) gemäß einem der vorhergehenden Ansprüche, wobei das Hauptlumen (712; 1612) in Fluidverbindung mit einem Fluiddurchgang zwischen dem ersten und dem zweiten Schaft (104; 105, 106) steht, welcher wiederum in Fluidverbindung mit dem Ballon (108) steht, so dass ein in das Aufpumpanschlusslumen eingeführtes Aufpumpfluid durch das Hauptlumen (712; 1612), den Fluiddurchgang und in den Ballon (108) strömen kann, um den Ballon (108) aufzupumpen.

11. Zuführvorrichtung (100; 1400) gemäß Anspruch 10, wobei die Aufpumpanschlussanordnung (700; 700'; 1600) so konfiguriert ist, dass ein Abziehen des Aufpumpfluids aus dem Ballon (108) über den Aufpumpanschluss bewirkt, dass einen Unterdruck im Aufpumpverteiler (706; 1602) herzustellen, der den Kolben (708; 1618) und den ersten Schaft (106) in distaler Richtung bewegt.

12. Zuführvorrichtung (100; 1400) gemäß einem der vorhergehenden Ansprüche, ferner umfassend ein Vorspannelement (1402; 1450), das dazu konfiguriert ist, den ersten Schaft (106) für eine Bewegung in distaler Richtung relativ zum zweiten Schaft (104; 105) vorzuspannen.

13. Zuführvorrichtung (100; 1400) gemäß Anspruch 12, wobei das Vorspannelement (1402; 1450) eine Feder (1402; 1450) umfasst.

14. Zuführvorrichtung (100; 1400) gemäß Anspruch 13, wobei die Feder (1402; 1450) um den ersten Schaft (106) herum innerhalb des Ballons (108) oder um den Kolben (708; 1618) herum angeordnet ist.

15. Zuführvorrichtung (100; 1400) gemäß einem der vorhergehenden Ansprüche, wobei der Hauptkörper (602) des Aufpumpverteilers (706; 1602) im Griff (102) angeordnet ist.

## Revendications

1. Appareil de pose (100 ; 1400) pour un dispositif prothétique implantable (10 ; 50 ; 114), l'appareil de pose (100 ; 1400) comprenant :
une poignée (102) ;
une première tige (106) s'étendant de manière distale à partir de la poignée (102) ;
une seconde tige (104 ; 105) s'étendant de manière distale à partir de la poignée (102), la première tige (106) s'étendant à travers la seconde tige (104 ; 105) et étant mobile axialement par rapport à la seconde tige (104 ; 105) ;
un ballonnet gonflable (108) présentant une partie extrémité proximale (126) et une partie extrémité distale (128), la partie extrémité proximale (126) du ballonnet (108) étant accouplée à une partie extrémité distale (152) de la seconde tige (104 ; 105) et la partie extrémité distale (128) du ballonnet (108) étant accouplée à une partie extrémité distale (112) de la première tige (106) ; et
un ensemble embout de gonflage (700 ; 700' ; 1600) comprenant un collecteur de gonflage (706 ; 1602) et un piston (708 ; 1618), le collecteur de gonflage (706 ; 1602) comprenant un corps principal définissant une lumière principale (712 ; 1612) s'étendant à travers celui-ci et
un orifice de gonflage définissant une lumière d'orifice de gonflage en communication fluidique avec la lumière principale (712 ; 1612), le piston (708 ; 1618) s'étendant dans la lumière principale (712 ; 1612) et pouvant coulisser par rapport au collecteur de gonflage (706 ; 1602) ;
une partie extrémité proximale de la première tige (106) étant accouplée au piston (708 ; 1618) et une partie extrémité proximale de la seconde tige (104 ; 105) étant accouplée au corps principal du collecteur de gonflage (706 ; 1602) ou à la poignée (102) ;
le piston (708 ; 1618) étant mobile par rapport au collecteur de gonflage (706 ; 1602) dans des sens proximal et distal de telle sorte qu'un déplacement distal du piston (708 ; 1618) produit un déplacement distal de la première tige (106) par rapport à la seconde tige (104 ; 105), ce qui augmente la longueur du ballonnet (108), et de telle sorte qu'un déplacement proximal du piston (708 ; 1618) produit un déplacement proximal de la première tige (106) par rapport à la seconde tige (104 ; 105), ce qui diminue la longueur du ballonnet (108).

2. Appareil de pose (100 ; 1400) selon la revendication 1, le piston (708; 1618) comprenant une tête (902) de piston et une tige (904) de piston, la tête (902) de piston comprenant une rainure annulaire (903) et un élément d'étanchéité annulaire (726) étant disposé dans la rainure annulaire (903), l'élément d'étanchéité (726) établissant un joint d'étanchéité contre une surface interne de la lumière principale (712 ; 1612) du collecteur de gonflage (706 ; 1602).

3. Appareil de pose (100 ; 1400) selon l'une quelconque revendication précédente, la partie extrémité proximale de la première tige (106) s'étendant dans une lumière (915) du piston (708 ; 1618) et étant fixée à l'intérieur de celle-ci.

4. Appareil de pose (100 ; 1400) selon l'une quelconque revendication précédente, la lumière principale (712 ; 1612) du collecteur de gonflage (706 ; 1602) comportant une paroi interne faisant saillie radialement qui est façonnée pour limiter le déplacement distal du piston (708 ; 1618) à l'intérieur de la lumière principale (712 ; 1612).

5. Appareil de pose (100 ; 1400) selon l'une quelconque revendication précédente, comprenant en outre un élément capuchon (730) disposé sur une partie extrémité proximale du corps principal (602) du collecteur de gonflage (706 ; 1602), l'élément capuchon (730) comprenant une saillie (748) positionnée pour limiter le déplacement proximal du piston (708 ; 1618) à l'intérieur de la lumière principale (712 ; 1612), la saillie (748) s'étendant de préférence radialement dans la lumière principale (712 ; 1612) du collecteur de gonflage (706 ; 1602).

6. Appareil de pose (100 ; 1400) selon la revendication 5, la saillie (748) comprenant une nervure (1002) qui s'étend dans une rainure (903) s'étendant axialement formée sur une surface externe du piston (708 ; 1618), la nervure (1002) empêchant la rotation du piston (708 ; 1618) par rapport à l'élément capuchon (730).

7. Appareil de pose (100 ; 1400) selon la revendication 5 ou 6, l'élément capuchon (730) formant une liaison par encliquetage avec la partie extrémité proximale du corps principal (602) du collecteur de gonflage (706 ; 1602).

8. Appareil de pose (100 ; 1400) selon la revendication 7, l'élément capuchon (730) comprenant une pluralité d'ouvertures (1004) et la partie extrémité proximale du corps principal (602) du collecteur de gonflage (706 ; 1602) comprenant une pluralité de saillies (748) dimensionnées pour s'étendre dans les ouvertures (1004) afin de former la liaison par encliquetage.

9. Appareil de pose (100 ; 1400) selon l'une quelconque des revendications 1 à 4, comprenant en outre un élément capuchon (730) disposé sur une partie extrémité proximale du corps principal (602) du collecteur de gonflage (706 ; 1602), l'élément capuchon (730) étant conçu pour permettre un déplacement axial du piston (708 ; 1618) et de la première tige (106) par rapport à l'élément capuchon (730) dans les sens proximal et distal et pour résister au mouvement de rotation du piston (708 ; 1618) et de la première tige (106) par rapport à l'élément capuchon (730).

10. Appareil de pose (100 ; 1400) selon l'une quelconque revendication précédente, la lumière principale (712 ; 1612) étant en communication fluidique avec un trajet de fluide entre les première et seconde tiges (104 ; 105, 106), qui est à son tour en communication fluidique avec le ballonnet (108) de telle sorte qu'un fluide de gonflage introduit dans la lumière d'orifice de gonflage peut s'écouler à travers la lumière principale (712 ; 1612), le trajet de fluide et dans le ballonnet (108) afin de gonfler le ballonnet (108).

11. Appareil de pose (100 ; 1400) selon la revendication 10, l'ensemble embout de gonflage (700 ; 700' ; 1600) étant conçu de telle sorte que le retrait du fluide de gonflage à partir du ballonnet (108) par l'intermédiaire de l'orifice de gonflage est efficace pour l'établissement d'un vide dans le collecteur de gonflage (706 ; 1602) qui déplace le piston (708 ; 1618) et la première tige (106) dans le sens distal.

12. Appareil de pose (100 ; 1400) selon l'une quelconque revendication précédente, comprenant en outre un élément de sollicitation (1402 ; 1450) conçu pour solliciter la première tige (106) à se déplacer dans le sens distal par rapport à la seconde tige (104 ; 105).

13. Appareil de pose selon la revendication 12, l'élément de sollicitation (1402 ; 1450) comprenant un ressort (1402 ; 1450).

14. Appareil de pose (100 ; 1400) selon la revendication 13, le ressort (1402 ; 1450) étant disposé autour de la première tige (106) à l'intérieur du ballonnet (108) ou autour du piston (708 ; 1618).

15. Appareil de pose (100 ; 1400) selon l'une quelconque revendication précédente, le corps principal (602) du collecteur de gonflage (706 ; 1602) étant disposé dans la poignée (102).
